(19) Europäisches Patentamt
European Patent Office
Office européen des brevets

(11) **EP 2 420 183 B1**

(12) **EUROPEAN PATENT SPECIFICATION**

(45) Date of publication and mention
of the grant of the patent:
**05.10.2016 Bulletin 2016/40**

(51) Int Cl.:
*A61B 5/117* (2006.01)       *A61B 5/145* (2006.01)
*G06F 19/00* (2011.01)       *G06Q 50/24* (2012.01)
*A61B 5/00* (2006.01)

(21) Application number: **10764406.4**

(22) Date of filing: **09.04.2010**

(86) International application number:
**PCT/JP2010/056465**

(87) International publication number:
**WO 2010/119822 (21.10.2010 Gazette 2010/42)**

(54) **USER-SPECIFIC DATA PROVISION SYSTEM, USER-SPECIFIC DATA PROVISION METHOD AND SERVER DEVICE**

BENUTZERSPEZIFISCHES DATENBEREITSTELLUNGSSYSTEM, BENUTZERSPEZIFISCHES DATENBEREITSTELLUNGSVERFAHREN UND SERVER

SYSTÈME D'APPROVISIONNEMENT EN DONNÉES SPÉCIFIQUES À UN UTILISATEUR, PROCÉDÉ D'APPROVISIONNEMENT EN DONNÉES SPÉCIFIQUES À UN UTILISATEUR ET DISPOSITIF DE SERVEUR

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **17.04.2009   JP 2009101291**
**27.10.2009   JP 2009246936**

(43) Date of publication of application:
**22.02.2012   Bulletin 2012/08**

(73) Proprietor: **ARKRAY, Inc.**
**Minami-ku**
**Kyoto-shi**
**Kyoto 601-8045 (JP)**

(72) Inventors:
• **KATSUKI, Koji**
**Kyoto-shi**
**Kyoto 6020008 (JP)**

• **SHIONOYA, Go**
**Kyoto-shi**
**Kyoto 602-0008 (JP)**
• **IKETANI, Kazuya**
**Kyoto-shi**
**Kyoto 602-0008 (JP)**

(74) Representative: **MacDougall, Alan John Shaw**
**Mathys & Squire LLP**
**The Shard**
**32 London Bridge Street**
**London SE1 9SG (GB)**

(56) References cited:
**JP-A- 2004 129 777       JP-A- 2006 023 973**
**JP-A- 2006 323 468       US-A1- 2005 075 116**
**US-A1- 2008 269 630**

**Description**

Technical Field

The present invention relates to a user-specific data (information) provision system for providing information held specifically by a user himself or herself, a user-specific data (information) provision method, a server device, a medical facility terminal and a handheld device.

Background Art

There is proposed a conventional system receiving blood glucose level information transmitted from a handheld type blood glucose level measuring device via an access point and managing the received blood glucose level information by a server. The system providing proper medical information to a user when abnormality in a state of the blood glucose occurs (refer to, e.g., Patent document 1). Further, it is proposed that a technology for continuously monitoring a concentration of the in-vivo blood glucose level of a human being and an animal, calculating the blood glucose level concentration which changes with time by a predetermined function expression and predicting a prospective blood glucose level concentration (refer to, e.g., Patent document 2).

Documents of Prior Arts

Patent Documents

Patent document 1: Japanese Patent Application Laid-Open Publication No. 2003-057244
Patent document 2: Japanese Patent Application Laid-Open Publication No. 2005-308742

Further background art is provided in US 2005/075116 A1, which discloses a wireless virtual campus escort system whereby a user selects a route between a starting point and a destination. A mobile handset monitors the user's progress along the route by monitoring the location and movement of the handset and, optionally, sensor data, and by comparing this information to rules that define permitted or prohibited locations or movements or threshold sensor values. The handset uses one or more positioning systems, such as GPS, to ascertain its location. A server provides the handset with information to correct errors in the location information. If a rule fires, possibly indicating that the user is in danger, the handset attempts to ascertain the user's wellbeing, warns the user to return to the prescribed route and begins sending the handset's location to a server, which displays the information to a dispatcher who dispatches safety or security personnel to the user's location. The handset and servers communicate via available wireless channel(s).
This document discloses a device according to the preamble of claim 1.

Summary of the Invention

Problems to be solved by the Invention

When a user falls into a state of unconsciousness such as a comatose state due to hypoglycaemia and is carried to a medical facility, a medical worker is not able to acquire information that is specifically held by the user himself or herself (which will hereinafter be referred to as user-specific information) from the user, and it is therefore difficult to implement a proper medical care, a proper diagnosis and a proper dosage of medicine matching with the user. The user-specific information is exemplified by, e.g., contents and amounts of the meals in recent one week (e.g., an amount of ingested carbohydrate), hours of sleep, hours of exercise, a daily-ingested medicine, an ingestion frequency thereof, anamnesis, a medical history, a chronic disease, allergy, etc. Especially when the user moves to a remote place or when moving to an absolutely strange place to which the user has never moved, there is no medical worker who knows the user very well in the medical facility in that region in many cases. It is therefore much more difficult for the medical worker to obtain the user-specific information if such a problem arises.
Moreover, according to a method of previously sending the user-specific information to the medical facility in a destination of the movement and using this user-specific information when the user undergoes a medical examination, large loads occur on both of the user and the medical facility. Namely, the user must search for the adequate medical facility located within the region in the destination of the movement and must send the user-specific information each time the user moves. Moreover, thereafter, in the case of further moving into another region, the user-specific information sent to the medical facility located in the previous region becomes unnecessary, and the user must ask the medical facility to erase this information. On the other hand, the medical facility is required to manage the user-specific information so as not to be used for purposes other than the medical care, the diagnosis and the dosage of medicine. Furthermore, if the user does not undergo the medical examination, the user-specific information is not effectively utilized, and as a result there increases a working load only for managing the user-specific information.

Further, according to a method by which the user records the user-specific information on a notebook etc beforehand and presents the notebook to the medical worker when undergoing the medical examination in the medical facility, the user himself or herself has a necessity of always carrying the recorded notebook, which accompanies a risk of being lost at all times. Moreover, in the case of forgetting to carry the notebook when going out, the user can not convey exactly the user-specific information to the medical worker when undergoing the medical examination.

It is an object of the present invention, which was devised in view of the problems described above, to get a medical facility to grasp properly the user-specific information without increasing the loads on the medical facility and on the user.

Means for Solving the Problems

The present invention is a user-specific information provision system as defined in Claim 1 of the appended claims. Also provided is a method as defined in Claim 5 and a server device as defined in Claim 9.

Respective modes of the present invention adopt the following configurations in order to solve the problems described above.

To accomplish the object, a user-specific information provision system according to an embodiment of the invention includes: a handheld device transmitting, to a server device, user-specific information held specifically by a user and user's biometric information acquired by measuring biometric information of the user; the server device receiving the user-specific information and the user's biometric information; and a medical facility terminal receiving information transmitted from the server device, the server device including an access point information determining unit acquiring access point information representing an access point to which the handheld device belongs at present and determining whether the access point is changed or not, at least one of the handheld device and the server device further including a biometric information prediction determining unit determining whether or not the user's biometric information satisfies a predetermined criterion (criteria), wherein the server device transmits the user-specific information to the medical facility terminal when both the access point information determining unit determines that the access point is changed and the biometric information prediction determining unit determines that the predetermined criterion is satisfied.

Further, to accomplish the object, an embodiment of the invention provides a user-specific information provision method executed by a server capable of receiving, from a handheld device, user-specific information held specifically by a user and user's biometric information acquired by measuring biometric information of the user, the server device receiving the user-specific information and the user's biometric information, the server device being capable of transmitting information to a medical facility terminal, the method including: a step of the server device acquiring access point information representing an access point to which the handheld device belongs at present and determining whether the access point is changed or not; a biometric information prediction determining step of the server device determining whether the user's biometric information satisfies a predetermined criterion or not, or the server device receiving from the handheld device the result of a determination made by the handheld device as to whether the user's biometric information satisfies a predetermined criterion or not; an access point information determining step of the server device determining whether the access point is changed or not; and a step of the server device transmitting the user-specific information to the medical facility terminal when it is determined that both the predetermined criterion is satisfied and the access point is changed.

Still further, to accomplish the object, an embodiment of the invention provides a server device receiving user-specific information transmitted from a handheld device and held specifically by a user and user's biometric information acquired by measuring biometric information of the user, including: an access point information determining unit acquiring access point information representing an access point to which the handheld device belongs at present and determining whether the access point is changed or not; a biometric information prediction determining unit determining whether or not the user's biometric information satisfies a predetermined criterion; and a medical facility terminal specifying unit transmitting the user-specific information to a medical facility terminal when it is determined that both the access point is changed and the predetermined criterion is satisfied.

Yet further, to accomplish the object, a handheld device is disclosed that is capable of transmitting, to a server device, user-specific information held specifically by a user and user's biometric information acquired by measuring biometric information of the user, the handheld device including: a biometric information prediction determining unit determining whether the biometric information satisfies a predetermined criterion or not; and a determination result transmitting unit transmitting a result of the determination to the server device when determining that the predetermined criterion is satisfied.

Moreover, to accomplish the object, a program is disclosed for making a computer execute: a process of receiving user-specific information transmitted from a handheld device and held specifically by a user and user's biometric information acquired by measuring biometric information of the user; a process of acquiring access point information representing an access point to which the handheld device belongs at present; a process of determining whether the access point is changed or not; a process of determining whether or not the user's biometric information satisfies a predetermined criterion; and a process of transmitting the user-specific information to the medical facility terminal when determining that the access point is changed and/or when determining that the predetermined criterion is satisfied.

According to these respective modes, even if the user falls into a comatose state due to, e.g., hypoglycaemia and reaches a state of being disabled from making mutual communications with the medical facility, the medical facility in which to install the medical facility terminal can properly grasp the sent user-specific information and, by extension, can implement a proper medical care or a proper diagnosis or a proper dosage of medicine for the user. Furthermore, new loads do not occur on both of the user and the medical facility because of obtaining these effects.


Effects of the Invention


According to the respective modes of the present invention, the medical facility is enabled to grasp properly the user-specific information without increasing the loads on the medical facility and the user.


Brief Description of the Drawings


FIG. 1A is a block diagram showing an architecture of a whole use-specific information provision system 100 according to an embodiment of the present invention.
FIG. 1B is an explanatory diagram of a handheld device 110 according to the embodiment of the present invention.
FIG. 1C is an explanatory diagram of a server device 140 according to the embodiment of the present invention.
FIG. 1D is an explanatory diagram of a medical facility terminal 170 according to the embodiment of the present invention.
FIG. 2A is a diagram illustrating a data record stored in an auxiliary storage unit of the handheld device.
FIG. 2B is a diagram illustrating a database stored in an auxiliary storage unit of the server device.
FIG. 2C is a diagram illustrating the database stored in the auxiliary storage unit of the server device.
FIG. 2D is a diagram illustrating the database stored in the auxiliary storage unit of the server device.
FIG. 2E is a diagram illustrating the database stored in the auxiliary storage unit of the server device.
FIG. 2F is a diagram illustrating a database stored in an auxiliary storage unit of the medical facility terminal.
FIG. 2G is a diagram illustrating the database stored in the auxiliary storage unit of the medical facility terminal.
FIG. 2H is a diagram illustrating a database that may be stored in the handheld device.
FIG. 3 is a flowchart illustrating a biometric information profile generation process of generating a user-specific biometric information profile.
FIG. 4 is a flowchart showing a method of calculating a predictive value of prospective user's biometric information by use of the user-specific biometric information profile generated based on a past user's blood glucose level data train and determining by use of this predictive value whether the user's biometric information satisfies a predetermined criterion or not.
FIG. 5 is a diagram showing one example of fluctuation patterns (patterns 1 - 6) of the past biometric information.
FIG. 6 is a flowchart showing a method of updating the user-specific information stored in a user-specific information storage unit within the server device.
FIG. 7 is a flowchart showing a method of how the server device transmits the user-specific information to the necessary medical facility terminal.
FIG. 8 is a flowchart showing a method of how the server device erases the user-specific information stored in the user-specific information storage unit within the medical facility terminal.


Mode for Carrying out the Invention


(Embodiment)


A detailed description of an embodiment of the present invention will hereinafter be made with reference to the drawings. Note that the present embodiment will have a discussion by exemplifying a case of continuously measuring a blood glucose level as biometric information of a user (hereinafter referred to as user's biometric information). However, the biometric information to be measured is not limited to the blood glucose level, and available pieces of biometric information are GOT, GPT, LDH, $\gamma$-GT, alkaline phosphatase, choline esterase, lipase, creatinine-kinase and ammonia.

Further, a measurement target is not limited to blood, and the measurement target may be blood sera, blood plasma, etc. Moreover, the measuring method, without being limited to the continuously measuring method, may also be a method of making the measurement per given period of time.

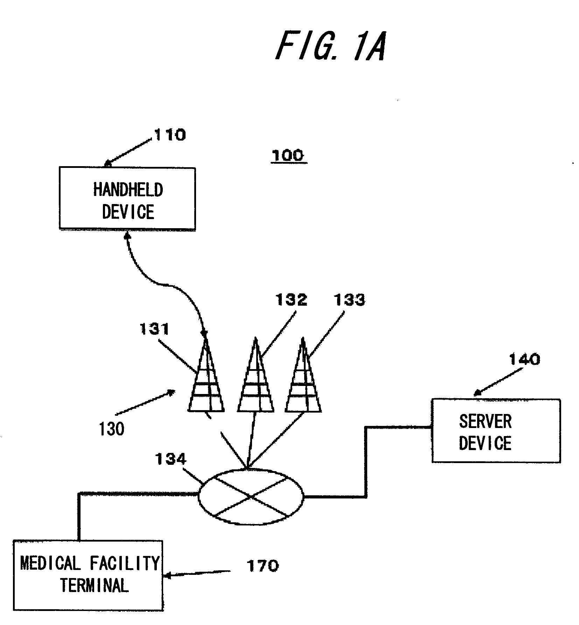

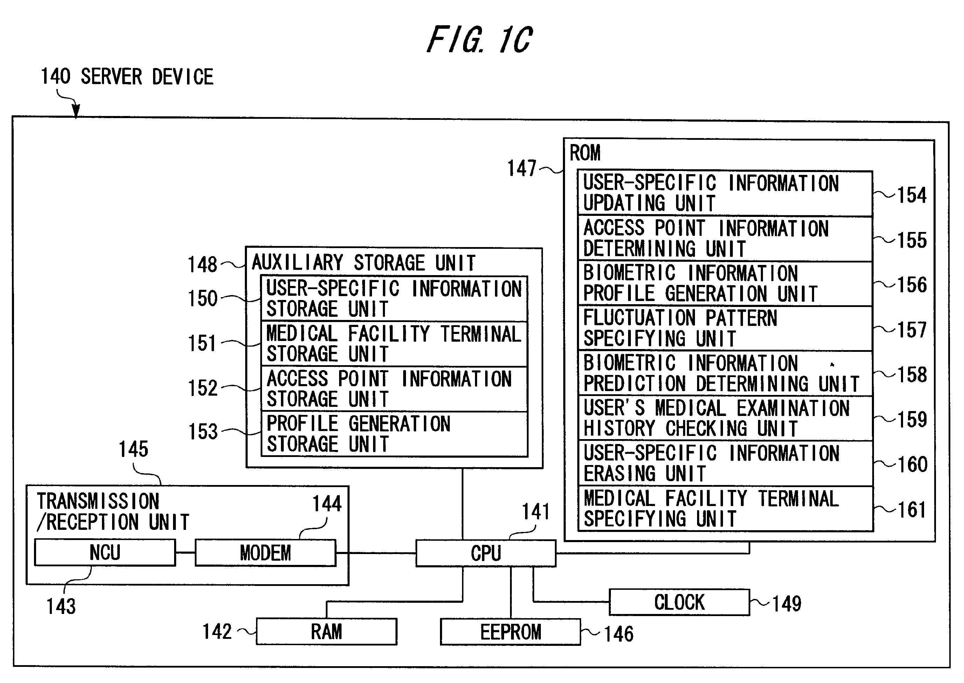

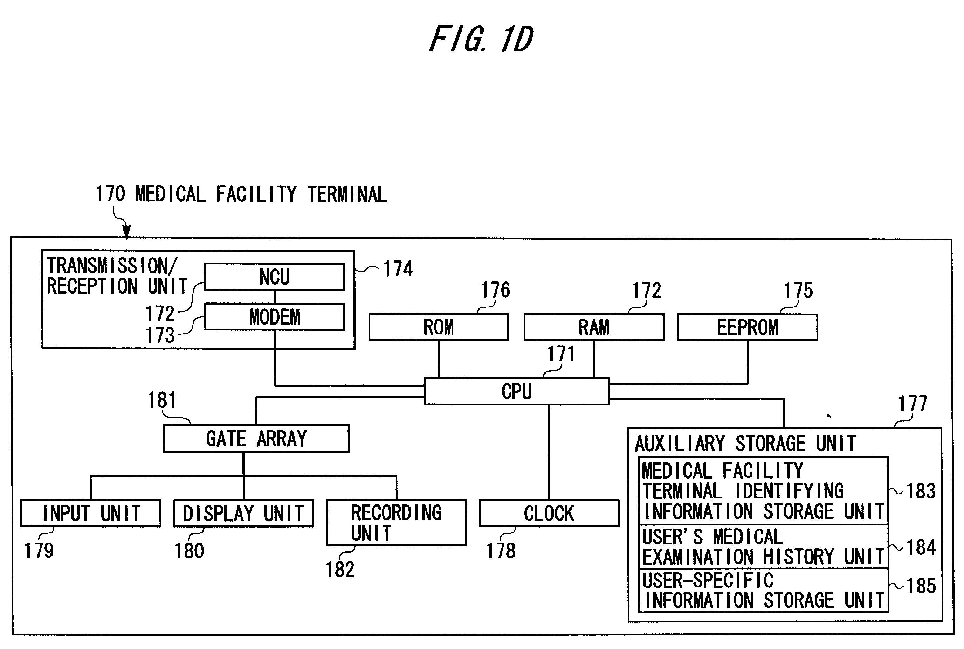

FIG. 1A is a block diagram showing a whole configuration of a user-specific information provision system 100 according to the embodiment of the present invention. The user-specific information provision system 100 is configured by a variety of devices such as a handheld device 110, access points 130, a server device 140 and a medical facility terminal 170. Configurations and characteristics of the respective devices will be described as follows.

The access points 130 are put in respective regions and communication-enabled ranges (regions) with the handheld devices 110 are set up, respectively. Then, the handheld device 110, which will be described later on, is coupled to one access point (an access point 131 in FIG. 1A) via the server device 140. The connection between the access point 130 and the handheld device 110 may be established by either a wireless system or a wired system. It is assumed that the handheld device 110 belongs to an access point 130 when the handheld device stays in the communication-enabled range with the access point 130. The connection between the access point 130 and the server device 140 may be established via a communication network 134 (such as a telephone network, the Internet and a satellite channel) and may also be established directly. Further, the access point 130 is provided with an information attaching unit which links and attaches access point information to information transmitted via the access point. The access point information is defined as information to identify individual access point, and is expressed by, for example, a number, a symbol, and the like. Note that the three access points, the access point 131, the access point 132 and the access point 133 exist in FIG. 1A, however, the number of the access points 130 is not limited to "3", and some other plural number of access points 130 may be adopted.

The handheld device 110 includes, as will hereinafter be explained, configuration of a computer. The handheld device 110 includes a CPU 111 which controls the handheld device 110 on the whole. The CPU 111 executes a variety of processes in accordance with instructions of various programs stored in a RAM 118. A measuring unit 112, an analog signal processing unit 113 which amplifies an output signal of the measuring unit 112, an A/D conversion unit 114 which converts the output signal given from the analog signal processing unit 113 into a digital signal, a transmission/reception unit 115 which transmits and receives the data to and from the outside, an auxiliary storage unit 116, a ROM 117 storing the various programs, the RAM 118 storing various items of data processed by the CPU 111 and the programs stored in the ROM 117, an EEPROM 119 storing flags and the like, a clock 120, a gate array 121 which controls an input/output (a display unit 122, an input unit 123) to/from the CPU 111, the display unit 122 and the input unit 123 connected to the gate array 121 are coupled to the CPU 111 via bus lines.

Moreover, the auxiliary storage unit 116 is further constructed of a user identifying information storage unit 124, a user-specific information storage unit 125, a measurement data storage unit 126 and a calibration curve data storage unit 127. The auxiliary storage unit 116 involves using a hard disk and a flash memory but is not limited to these devices. Note that the handheld device 110 is preferably a portable device which can be carried solely by the user but is not limited to this portable device. The measuring unit 112 detects an in-vivo change of the user as a signal. For example, as described in Japanese National Publication of International Patent Application No.2004-520898, a measuring device is exemplified that the signals representing the blood glucose levels are continuously captured by a blood glucose level sensor inserted into user's body, a skin of an arm region, an abdomen region, etc. via an insertion needle. Note that the measuring device is not limited to the above-mentioned measuring device. For example, as described in Japanese Publication of Examined Application No.Hei08-20412, a measuring device having a type using a disposable blood glucose level sensor is available. The disposable blood glucose level sensor detects a signal representing a blood glucose level by suction of blood bled on a surface of a fingertip, an abdomen region or an arm region by puncture thereof with a puncture needle performed per measurement. The signal representing the in-vivo change of the user, which is output from the measuring unit 112, is amplified by the analog signal processing unit 113, converted into the digital signal by the A/D conversion unit 114 and transmitted to the CPU 111.

The CPU 111 refers to the calibration curve data stored in the calibration curve data storage unit 127 of the auxiliary storage unit 116, then converts the output signal into a constituent concentration and displays a concentration value as measurement data on the display unit 122. In addition, the CPU 111 acquires date/time information from the clock 120 and stores the acquired date/time information and the measurement data associated with the acquired date/time information as user's biometric information in the measurement data storage unit 126. The transmission/reception unit 115 configuring a transmission/reception unit is coupled with a modem 128 and a communication circuit (NCU (Network Control Unit)) 129 and performs network controls. Further, the modem 128 demodulates the reception data and modulates the transmission data.

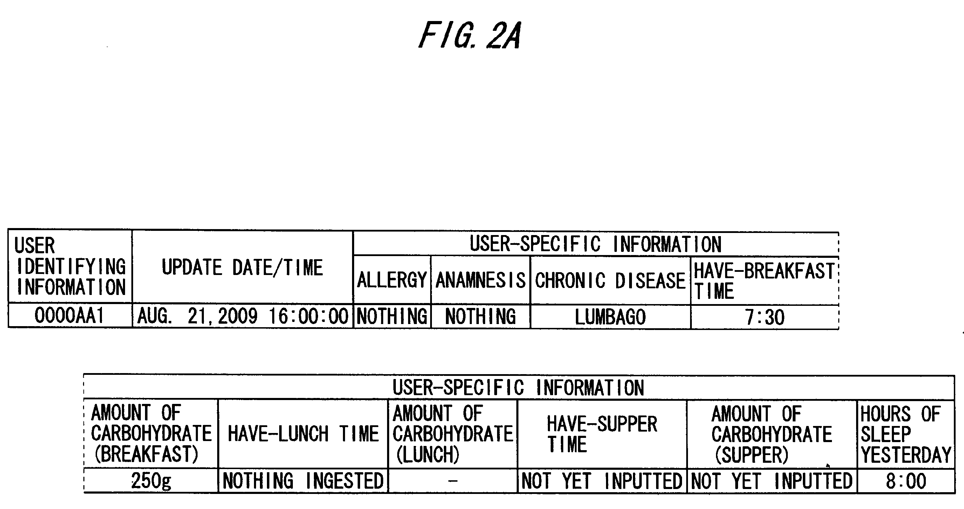

The user identifying information storage unit 124 stores with the user identifying information. Note that the user identifying information is a piece of information which enables identification of the user himself or herself using the handheld device or a piece of information enabling identification of the handheld device 110 itself, and is exemplified such as an ID number, a barcode, a QR code, a two-dimensional code and a manufacturing number. Note that a means for displaying the user identifying information on the handheld device 110 may take not only a mode of displaying the user identifying information on the display unit but also a mode of pasting the user identifying information onto the surface of a housing of the handheld device 110. The user-specific information storage unit 125 is one record that user-specific information, user identifying information of the handheld devices 110 and update time (which is the date/time information acquired from the clock) associated with the user-specific information is stored. FIG. 2A illustrates an example of this record. In the example of FIG. 2A, the user-specific information has contents of respective fields such as a "have-breakfast-time" field, a "have-lunch-time" field, a "have-supper-time" field, "amount-of-carbohydrate" fields for breakfast, lunch and supper, an "allergy" field, an "anamnesis" field and a "chronic disease" field but is not limited to these contents. The user-specific information may be updated corresponding to an option of the user, or alternatively such a mode may also be taken that the CPU 111 periodically generates an alarm on the display unit by use of the date/time information acquired from the clock 120 and prompts the user to perform these updating operations. If the user updates the data, upon inputting the update contents in the respective fields to the input unit, the CPU 111 refers to the fields of the user-specific information storage unit 125 and thus updates the data according to the update contents inputted to the input unit.

The server device 140 includes a configuration of the computer as will be described below. The server device 140 is equipped with a CPU 141 which controls the server device on the whole. The CPU 141 executes a variety of processes in accordance with instructions of various categories of programs stored in a RAM 142. Components connected via buses to the CPU 141 are a transmission/reception unit 145 constructed of a NCU 143 and a modem 144, an EEPROM 146 stored with flags and the like, a ROM 147 stores various programs, the RAM 142 stores various items of data processed by the CPU 141 and the programs stored in the ROM 147, an auxiliary storage unit 148 and a clock 149. The NCU 132 configuring a transmission/reception unit connects with the modem 144 and a communication circuit and performs network controls. Further, the modem 144 demodulates the reception data and modulates the transmission data.

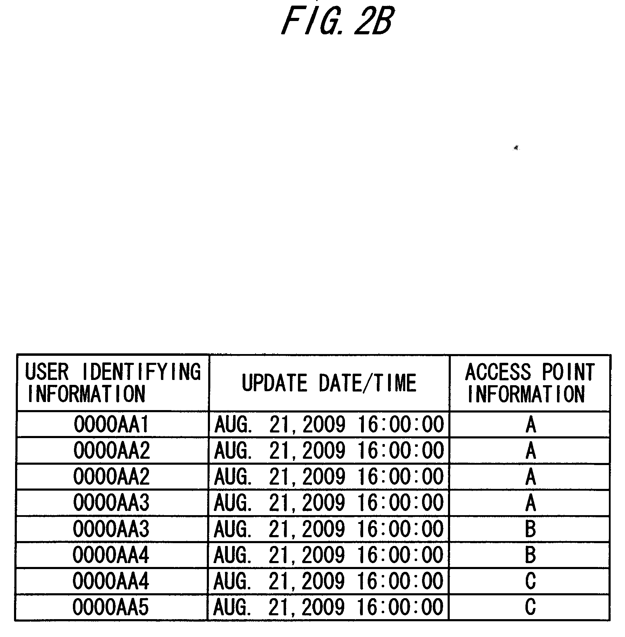

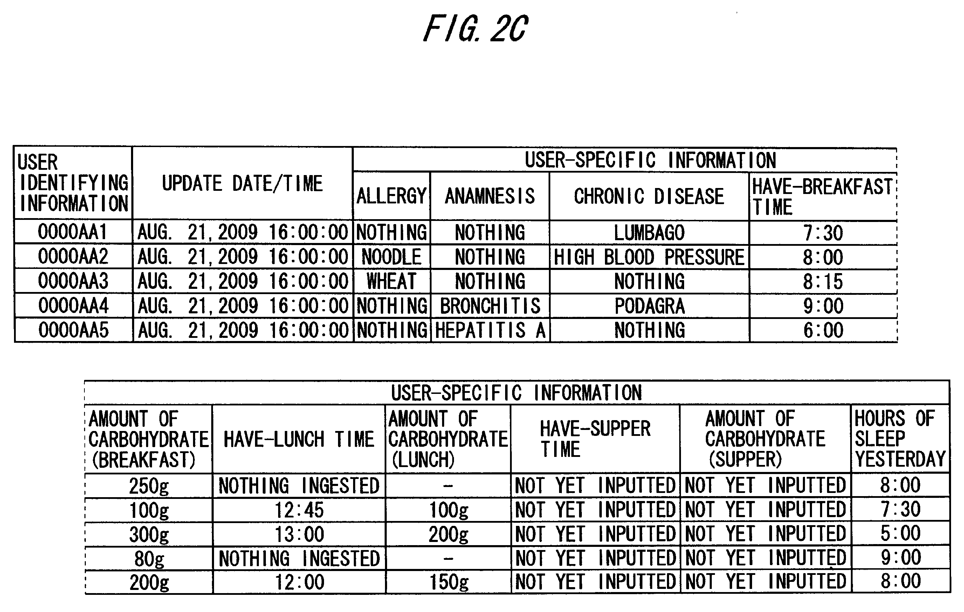

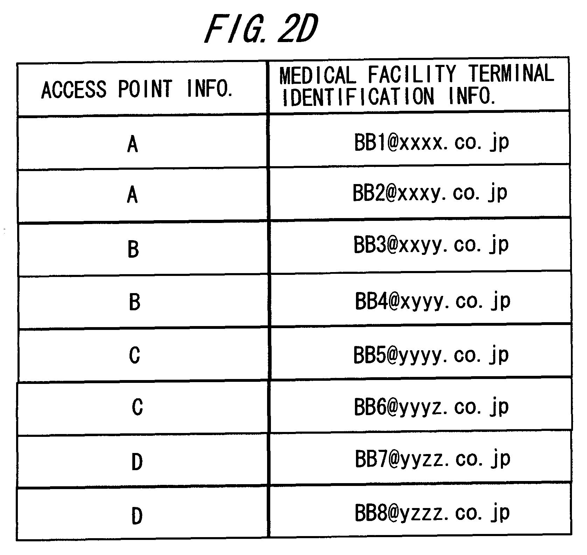

Moreover, the auxiliary storage unit 148 is further constructed of databases such as a user-specific information storage unit 150, a medical facility terminal storage unit 151, an access point information storage unit 152 and a profile generation storage unit 153. The auxiliary storage unit 148 involves using the hard disk and the flash memory but is not limited to these devices. The access point information storage unit 152 is the database in which the user identifying information of individual handheld device 110, the access point information and the update time (the date/time information acquired from the clock) are associated with each other in order to grasp the access point 130 to which each handheld device 110 belongs at the present. FIG. 2B illustrates an example of this database. The user-specific information storage unit 150 is the database in which pieces of user identifying information of the individual handheld devices 110, the update time (the date/time information acquired from the clock) and pieces of user-specific information of the users who use the individual handheld devices 110 are associated with each other. FIG. 2C illustrates this database. The medical facility terminal storage unit 151 is the database in which identifying information of the medical facility terminal that will be described later on and the access point information are associated each other in order for each access point 130 to grasp the medical facility terminal 170 located in the communication-enabled range with the handheld device 110. FIG. 2D shows this database. Note that each of the access points 131 - 133 does not necessarily have to store all of the medical facility terminals 170 located within the communication-enabled range with the handheld device 110 but may store at least one single medical facility terminal 170 which performs the most proper treatment in the region concerned.

Further, the ROM 147 is constructed of a user-specific information updating unit 154, an access point information determining unit 155, a biometric information profile generation unit 156, a fluctuation pattern specifying unit 157, a biometric information prediction determining unit 158, a user's medical examination history checking unit 159, a user-specific information erasing unit 160 and a medical facility terminal specifying unit 161. The user-specific information updating unit 154 is stored with a program enabling execution of a process of comparing the user-specific information transmitted from the handheld device 110 with the user-specific information stored in the user-specific information storage unit 150 and, if different, updating the user-specific information stored in the user-specific information storage unit 150 of the server device 140 into the user-specific information transmitted from the handheld device 110. The access point information determining unit 155 is stored with a program enabling the execution of a process of determining, based on the access point information periodically transmitted from the access point 130 and indicating the access point to which the individual handheld device belongs at the present, whether the belonging access point is changed or not and a process of updating, when changing the access point to which the handheld device 110 belongs, the access point information, stored in the access point information storage unit 152, of the handheld device 110 into the access point information indicating the now-belonging access point by referring to the access point information storage unit 152.

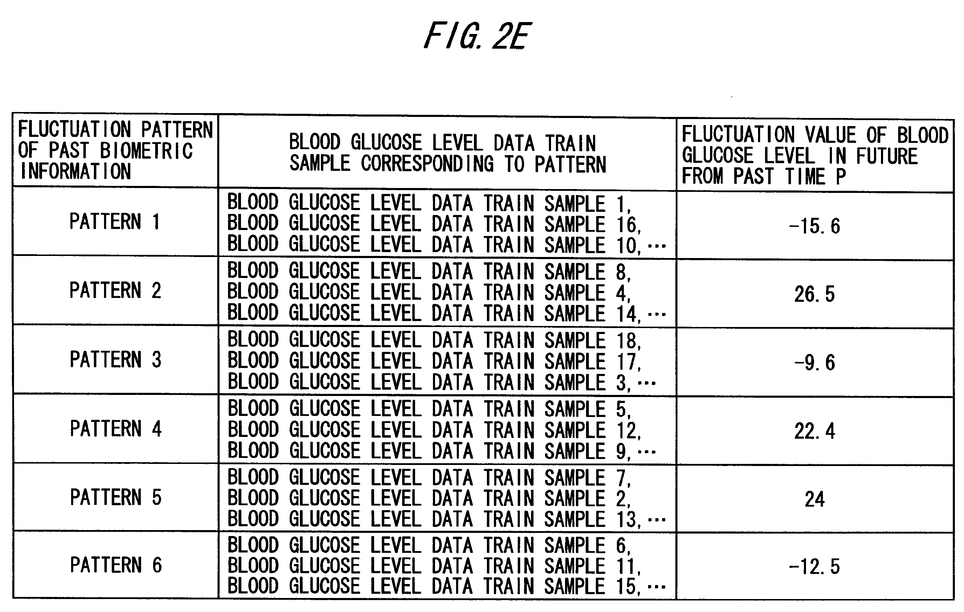

The biometric information profile generation unit 156 stores a program that is enabling execution of a process to generate a biometric information profile specific to the user based on a past user's blood glucose level data column (defined as user's biometric information transmitted in the past from the handheld device) accumulated at a predetermined time period, and to store the biometric information profile specific to the user, as a database (shown in FIG. 2E), in the profile generation storage unit 153 provided in the auxiliary storage unit 148. The fluctuation pattern specifying unit 157 stores a program that is enabling execution of a process to specify a fluctuation pattern of the past user's biometric information, which is coincident with a fluctuation pattern of user's recent biometric information, among the user-specific information profiles stored in the profile generation storage unit 153. The biometric information prediction determining unit 158 stores a program that is enabling execution of a process to calculate a predictive value of the prospective user's biometric information that is obtained by adding a fluctuation value of the prospective user's biometric information associated with the fluctuation pattern, specified by the fluctuation pattern specifying unit 157, of the past user's biometric information to the user's present biometric information, and to determine by using this predictive value of the prospective user's biometric information whether the user's biometric information satisfies a predetermined criterion or not.

The user's medical examination history checking unit 159 executes a process to determine whether or not the user undergoes the medical examination in the medical facility that the medical facility terminal 170, which will be described later on, is installed. To be specific, the user's medical examination history checking unit 159 stores a program that is enabling execution of a process to specify user's medical examination date/time stored in a user's reception history unit 184 provided in an auxiliary storage unit 177 of the medical facility terminal 170 that will be explained later on and to specify reception date/time of the user-specific information stored in a user-specific information storage unit 185 provided similarly in the auxiliary storage unit 177, and to determine, if the medical examination date/time is posterior to the reception date/time, that the user undergoes the medical examination. The user-specific information erasing unit 160 stores a program that is enabling execution of a process to erase the user identifying information stored in the user-specific information storage unit 185 provided in the auxiliary storage unit 177 of the medical facility terminal 170 that will be described later on, all items of information (the reception date/time, the user-specific information) associated with the respective pieces of user identifying information, the user identifying information stored in a user's medical examination history unit 184 provided similarly in the auxiliary storage unit 177 and the user's medical examination date/time associated with the user identifying information. The medical facility terminal specifying unit 161 stores a program that is enabling execution of a process to specify the medical facility terminal 170 from medical facility terminal recognizing information associated with access point information, which is stored in the medical facility terminal storage unit 151, indicating an access point to which individual handheld device 110 currently belongs, and to transmit the user-specific information to the thus-specified medical facility terminal 170.

The medical facility terminal 170 includes a configuration of the computer as will hereinafter be explained. The medical facility terminal 170 is equipped with a CPU 171 which controls the medical facility terminal 170 on the whole. The CPU 171 executes a variety of processes in accordance with instructions of various programs stored in a RAM 172. A transmission/reception unit 174 constructed of a NCU 172 and a modem 173, an EEPROM 175 storing flags and the like, a ROM 176 storing various programs, a RAM 172 storing various items of data processed by the CPU 171 and the programs stored in the ROM 176, an auxiliary storage unit 177, a clock 178, a gate array 181 which controls an input and an output (an input unit 179, a display unit 180) to/from the CPU 171, i.e., the display unit 180, the input unit 179 and a recoding unit 182 that are coupled to the gate array 181 are coupled to the CPU 171 via bus lines. The medical facility terminal 170 is provided within the medical facility. The medical facility is not limited to a permanent facility such as a hospital and a clinic. The medical facility may also be a movable facility such as an ambulance when taking account of a case where the user falls into a comatose state due to hypoglycaemia and is carried to the permanent facility such as the hospital and the clinic, during which the user-specific information is required. The NCU 172 configuring the transmission/reception unit 174 connects with the modem 173 and the communication circuit and performs network controls. Further, the modem 173 demodulates the reception data and modulates the transmission data. Moreover, the auxiliary storage unit 177 is further constructed of databases such as a medical facility terminal identifying information storage unit 183, a user's medical examination history unit 184 and a user-specific information storage unit 185. The auxiliary storage unit 177 involves using the hard disk and the flash memory but is not limited to these devices.

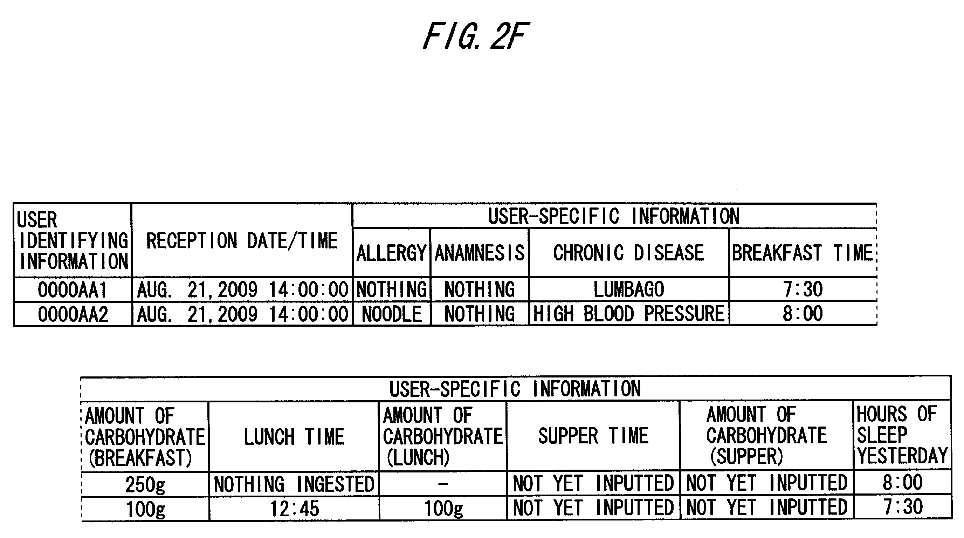

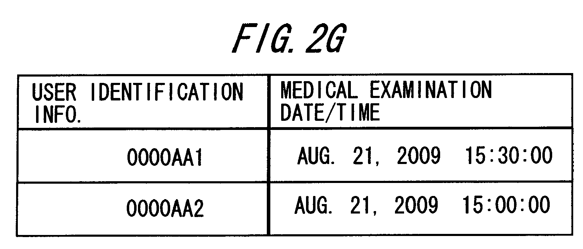

The medical facility terminal identifying information storage unit 183 stores the medical facility terminal identifying information. Note that the medical facility terminal identifying information is defined as the identifying information allocated to each medical facility terminal 170 and involves using, for example, a mail address of the medical facility terminal 170. The user-specific information storage unit 185 is a database in which pieces of user identifying information, which is received from the server device, of the individual handheld devices 110 and pieces of user-specific information are associated together with the reception date/time (the date/time information is acquired from the clock, hereinafter referred to as reception date/time) thereof. FIG. 2F illustrates this database. The user's medical examination history unit 184 is a database that the user identifying information inputted by the input unit 179 is associated with inputted date/time (the date/time information is acquired from the clock, hereinafter referred to as medical examination date/time). FIG. 2G illustrates this database. The input unit 179 is a device to input the user identification information, which is displayed on the display unit 112 of the handheld device 110 or is attached to the handheld device, to the medical facility terminal 170 when the user using the handheld device 110, to take medical examination, goes to the medical facility that the medical facility terminal 170 is installed or the user having the handheld device 110 is carried to the medical facility, and may also be a barcode-reader-based reading device and a device which inputs the user-specific information by a keyboard and the like. The display unit 180 displays the user identifying information and the user-specific information and is constructed of, for example, a liquid crystal screen and the like.

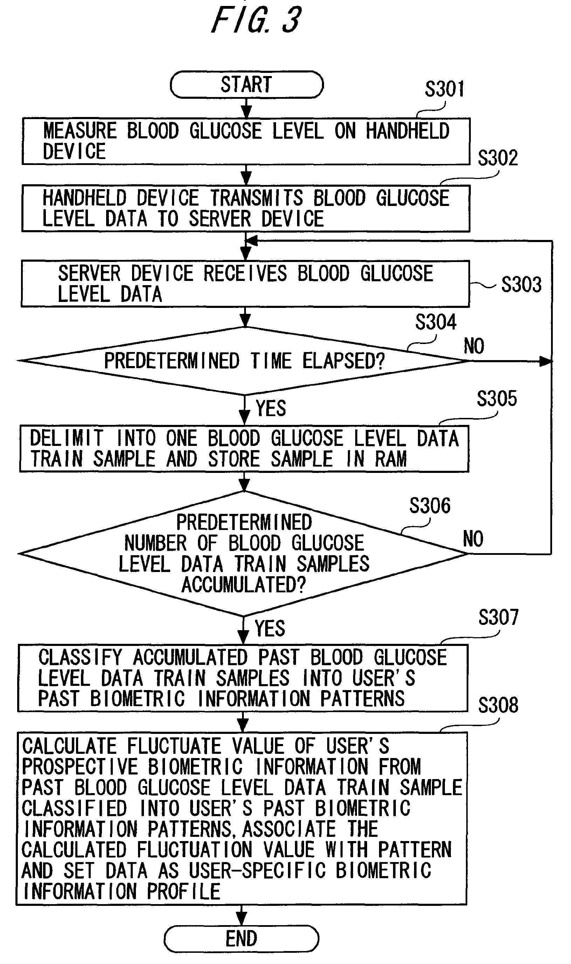

Explained next with reference to FIGS. 3, 4 and 5 is a method that the server device 140 or the handheld device 110 stores the user-specific information, then calculates the predictive value of the prospective user's biometric information and determines, by using it, whether the user's biometric information satisfies the predetermined criterion or not. Note that the discussion will be made by exemplifying a case of measuring the blood glucose level as the user's biometric information.

First, a biometric information profile generation process to generate a biometric information profile specific to the user will be described according to a flowchart in FIG. 3.

The measuring unit 112 of the handheld device 110 measures the blood glucose level of the user as the user's biometric information, and continuously acquires the blood glucose level data (step S301). The operation of acquiring the blood glucose level data may be performed based on an arbitrary instruction of the user and may also take a mode of being performed automatically periodically by the handheld device 110.

Next, the transmission/reception unit 115 of the handheld device 110 transmits, to the server device 140, the user's blood glucose level data that are continuously acquired by the measuring unit 112 (step S302). Subsequently, when the transmission/reception unit 145 of the server device 140 starts receiving the continuously-acquired blood glucose level data of the user, which are transmitted from the handheld device 110, the CPU 141 executes a process of reading the program stored in the biometric information profile generation unit 156 included in the ROM 147 into the RAM 142 and continuing to store the continuously-received blood glucose level of the user in the RAM 142 (step S303).

Next, the CPU 141, after starting continuously storing the blood glucose level data of the user in the RAM 142 in step S303, determines whether a predetermined period of time elapses or not (step S304). If it is determined that the predetermined period of time does not elapse (S304; NO), the CPU 141 loops the processing back to step S303. Whereas if it is determined that the predetermined period of time elapses (S304; YES), the CPU 141 delimits, as one blood glucose level data column sample, the user's blood glucose level data that are continuously stored in the RAM 142, and stores again (step S305).

Subsequently, the CPU 141 determines whether a predetermined number of blood glucose level data column samples are stored in the RAM 142 or not (step S306). When determining that the predetermined number of samples are not yet stored (S306; NO), the CPU 141 loops the processing back to step S303, and stores a new blood glucose level data column sample in the RAM 142 within the processes down to step S305. While on the other hand, when determining that the predetermined number of blood glucose level data column samples are stored therein (S306; YES), the CPU 141 proceeds the processing to step S307.

Next, in step S307, the CPU 141 executes at first a process of calculating a deviation rate of the blood glucose level, a variation rate of the blood glucose level and a difference of the blood glucose level shown in the blood glucose level data columns anterior to past time P with respect to each of the predetermined number of blood glucose level data column samples (representing the user's biometric information in the past). Note that the past time P represents the time P counted past from each point of the latest time, which is contained in each blood glucose level data column sample. Hereafter, it is shown that is a concrete calculation method of calculating the deviation rate of the blood glucose level, the variation rate of the blood glucose level and the difference of the blood glucose level.

The deviation rate of the blood glucose level is calculated by the following formula (1), where DATAp is a blood glucose level at the past time P, and DATAf is an average value of the blood glucose levels during a period of time having a predetermined length anterior A-hours to the past time P.

$$(DATAp - DATAf) / DATAf \times 100 \quad (Formula\ 1)$$

The variation rate of the blood glucose level is calculated by the following formula (2), where DATAk is an average value of the blood glucose levels during a period of time having a predetermined length anterior to the past time P, and DATAf is the average value of the blood glucose levels during a period of time having a predetermined length anterior A-hours to the past time P.

$$(DATAf - DATAk) / A \quad (Formula\ 2)$$

The difference of the blood glucose level is calculated by the following formula (3), where DATAp is the blood glucose level at the past time P, and DATAa is a blood glucose level anterior A-hours to the past time P.

$$DATAp - DATAa \quad (Formula\ 3)$$

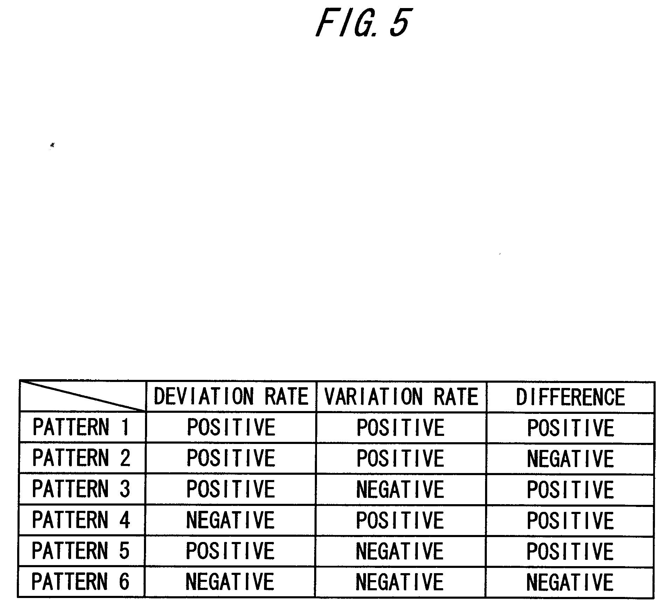

Next, the CPU 141 executes a process of calculating the deviation rate of the blood glucose level (Formula 1), the variation rate of the blood glucose level (Formula 2) and the past blood glucose level based on the difference of the blood glucose level (Formula 3) with respect to each of the predetermined number of stored blood glucose level data column samples as described above, and thereafter determining which pattern among patterns 1 - 6 (each representing a fluctuation pattern of the user's biometric information in the past) as illustrated in FIG. 5 that each of the blood glucose level data column samples is applied to (step S307). The CPU 141, after this determination process, proceeds the processing to step S308.

Next, in step S308, the CPU 141 transforms, into a frequency distribution, the fluctuation values of the blood glucose levels posterior X-hours to the past time P, which are indicated by the individual blood glucose level data column sample with respect to each of the patterns 1 - 6 (each representing a fluctuation pattern of the user's biometric information in the past) to which the blood glucose level data column sample is applied, and sets a median thereof as the fluctuation value of the blood glucose level in the future from the past time P (representing the fluctuation value of the user's biometric information in the future). Note that as for the calculation of the fluctuation values, other than the median described above, there may also be taken an average value of the fluctuation values of the blood glucose level posterior X-hours to the past time P. The CPU 141 generates the biometric information profile specific to the user, in which the thus-acquired fluctuation pattern of the past user's biometric information is associated with the fluctuation values of the blood glucose level in the future from the time P when that fluctuation pattern occurs (which represent the fluctuation values of the user's biometric information in the future), and stores the biometric information profile in a format of a database as illustrated in FIG. 2E in the profile generation storage unit 153 provided in the auxiliary storage unit 148 (step S308). Thereafter, the CPU 141 erases the program read from the biometric information profile generation unit 156 that is stored in the RAM 142, to terminate the processing.

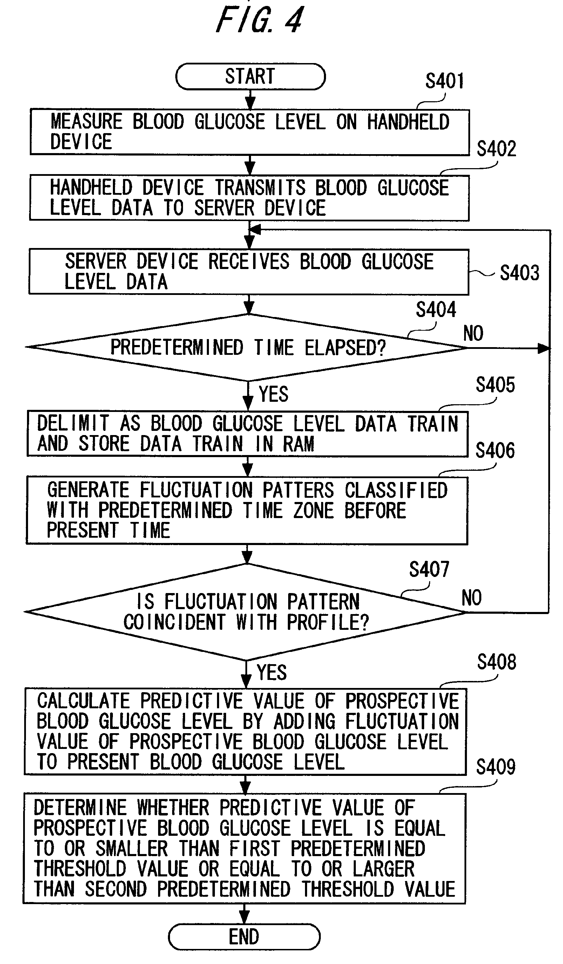

Explained next with reference to a flowchart in FIG. 4 is a method of calculating the predictive value of the prospective user's biometric information by using the user-specific biometric information profile generated based on the past user's blood glucose level data column as described above, and determining, by using it, whether the user's biometric information satisfies the predetermined criterion or not.

The measuring unit 112 of the handheld device 110 measures the blood glucose level of the user as the user's biometric information, and continuously acquires the blood glucose level data (step S401). The operation of acquiring the blood glucose level data may be performed based on the arbitrary instruction of the user and may also take the mode of being performed automatically periodically by the handheld device 110.

Next, the transmission/reception unit 115 of the handheld device 110 transmits, to the server device 140, the user's blood glucose level data that are continuously acquired by the measuring unit 112 (step S402). Subsequently, the transmission/reception unit 145 of the server device 140 starts receiving the continuously-acquired user's blood glucose level data transmitted from the handheld device 110, then the CPU 141 executes a process of reading the program stored in the fluctuation pattern specifying unit 157 provided in the ROM 147 into the RAM 142 and continuing to store the continuously-received user's blood glucose level data in the RAM 142 (step S403).

Next, the CPU 141 determines whether or not a predetermined period of time elapses since the start of continuously storing the user's blood glucose level data in the RAM 142 in step S403 (step S404). When determining that the predetermined period of time does not elapse, the CPU 141 loops the processing back to step S403. While on the other hand, when determining that the predetermined period of time elapses, the CPU 141 delimits, as one blood glucose level data column, the user's blood glucose level data that are continuously stored in the RAM 142, and stores in the RAM 142 again (step S405).

Next, in step S405, the CPU 141 generates the fluctuation pattern of the recent blood glucose levels (which represents the fluctuation pattern of the user's recent biometric information) based on the blood glucose level data column stored in step S405. To be specific, the CPU 141 generates the fluctuation pattern of the recent blood glucose levels (which represents the fluctuation pattern of the user's recent biometric information) including the deviation rate of the blood glucose level, the variation rate of the blood glucose level and the difference of the blood glucose level shown in the recent blood glucose level data column by the same calculation method as the method in step S307 (step S406).

Subsequently, the CPU 141 refers to the database (FIG. 2E) of the user-specific information profile stored in the profile generation storage unit 153 and thus determines whether or not the fluctuation pattern of the past user's biometric information is coincident with the fluctuation pattern of the user's recent biometric information (step S407). If determined not to be coincident in step S407 (S407; NO), the CPU 141 erases the blood glucose level data column stored in the RAM 142 and loops the processing back to step S401. Whereas if determined to be coincident in step S407 (S407; YES), the CPU 141 erases the program read from the fluctuation pattern specifying unit 157 that is stored in the RAM 142, and reads the program stored in the biometric information prediction determining unit 158 provided in the ROM 147 into the RAM 142. Then, the CPU 141 executes a process of referring to the database (FIG. 2E) of the user-specific biometric information profile and calculating the predictive value of the blood glucose level in the future (which represents the predictive value of the user's biometric information in the future) by adding the fluctuation value of the blood glucose level in the future from the time P (which represents the fluctuation value of the user's biometric information in the future) associated with the fluctuation pattern of the past user's biometric information coincident therewith to the present blood glucose level (step S408).

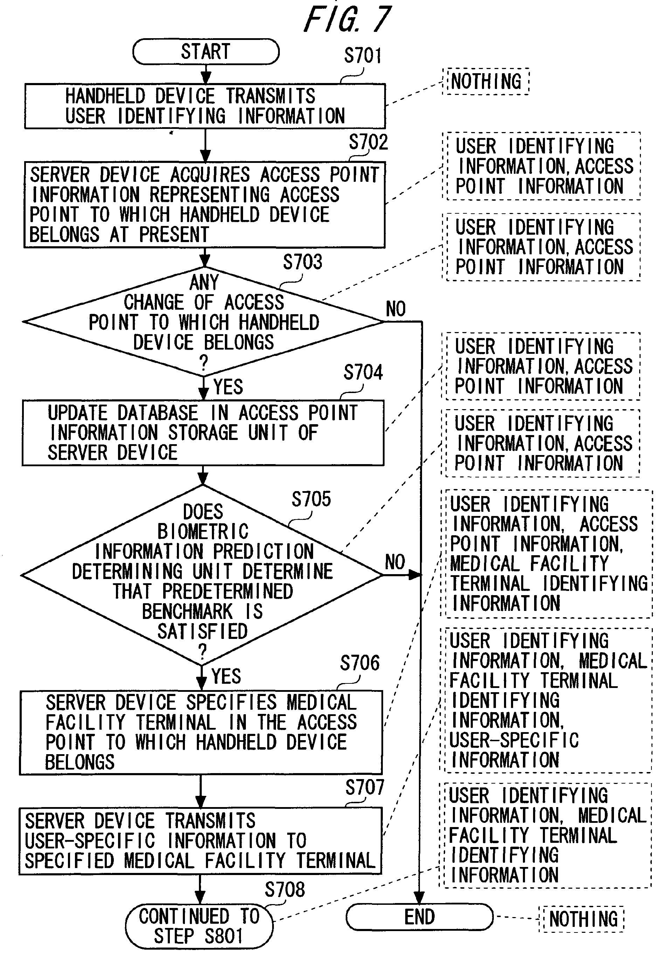

Next, the CPU 141 determines whether or not the calculated predictive value of the prospective blood glucose level is equal to or smaller than a first predetermined threshold value which is predetermined corresponding to the user, thereby determining whether the user will fall into the hypoglycaemic state or not. In this case, the CPU 141, if equal to or smaller than the first predetermined threshold value, deems that the user will fall into the hypoglycaemic state and therefore determines that the blood glucose level satisfies the predetermined criterion. Then, the CPU 141 erases the program read from the biometric information prediction determining unit 158 that is stored in the RAM 142, and advances the processing to step S706 (FIG. 7) as will be described later on. Alternatively, such a scheme may also be taken that the CPU 141 determines whether or not the calculated predictive value of the prospective blood glucose level is equal to or larger than a second predetermined threshold value which is predetermined corresponding to the user, thereby determining whether the user will fall into a hyperglycaemic state or not. In this case, the CPU 141, if equal to or larger than the second predetermined threshold value, deems that the user will fall into the hyperglycaemic state, and therefore determines that the blood glucose level satisfies the predetermined criterion. Then, the CPU 141 erases the program read from the biometric information prediction determining unit 158 that is stored in the RAM 142, and advances the processing to step S706 (FIG. 7) as will be described later on (step S409).

Further, the biometric information profile generation unit 156, the fluctuation pattern specifying unit 157 and biometric information prediction determining unit 158 are employed for predicting the prospective blood glucose level by using the calculation method described above and determining based on this whether the predetermined criterion is satisfied or not, however, the method is not limited to the calculation method described above. For example, the method of predicting the prospective blood glucose level may involve using a method described in Japanese Patent Laid-Open Publication No. 2005-308742.

Further, the determination method may involve not only the determination based on the above-calculated prospective blood glucose level but also the determination based on the present blood glucose level. For instance, an available scheme is that the biometric information prediction determining unit 158 sets the first predetermined threshold value that is predetermined corresponding to the user and determines, if the present blood glucose level is equal to smaller than the first predetermined threshold value, that the blood glucose level satisfies the predetermined criterion. Alternatively, there is taken another available scheme of setting the second predetermined threshold value, which is predetermined corresponding to the user, and, if the present blood glucose level is equal to or larger than the second predetermined threshold value, determining that the blood glucose level satisfies the predetermined criterion.

Moreover, the determination method may involve not only the determination based on the calculated prospective blood glucose level or based on the present blood glucose level but also the determination based on a rising speed or a lowering speed of the present blood glucose level (both of speeds are obtained from the present blood glucose level and the immediate blood glucose level). For example, an available scheme is that the biometric information prediction determining unit 158 sets a first speed threshold value which is predetermined corresponding to the user and, if the lowering speed of the present blood glucose level is equal to or smaller than the first speed threshold value, determines that the blood glucose level satisfies the predetermined criterion. Moreover, there may be taken another available scheme of setting the second predetermined threshold value which is predetermined corresponding to the user and, if the rising speed of the present blood glucose level is equal to or larger than this second predetermined threshold value, determining that the blood glucose level satisfies the predetermined criterion.

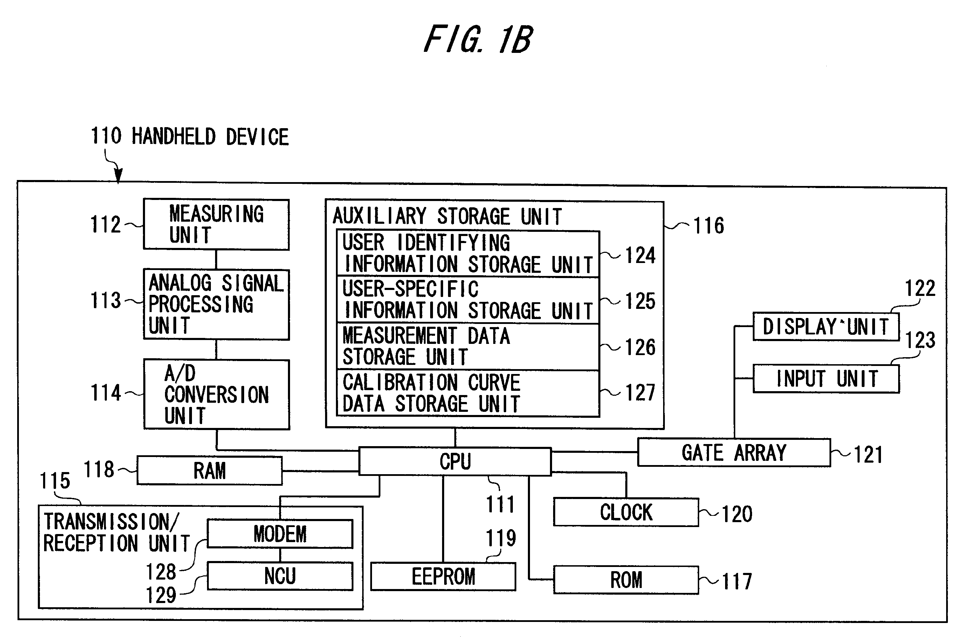

Incidentally, such a mode may also be adopted that the biometric information profile generation unit 156, the fluctuation pattern specifying unit 157, the biometric information prediction determining unit 158 and the profile generation storage unit 153 are stored in not the server device 140 but the ROM 117 of the handheld device 110 (the biometric information profile generation unit 156 is stored in the auxiliary storage unit 116 of the handheld device 110), and the CPU 111 of the handheld device 110 reads the various programs stored in the ROM 117 into the RAM 118 and executes the same process as the process of the CPU 141 of the server device 140 explained in FIGS. 3 and 4. In this case, the handheld device 110 further stores newly, in the ROM 117, a determination result transmitting unit (which is not illustrated in FIG. 1B) containing a program enabling the execution of a process of transmitting, if the user's biometric information is determined to satisfy the predetermined criterion in step S409, a result of being determined to satisfy the criterion to the server device 140. Then, if the user's biometric information is determined to satisfy the predetermined criterion in step S409, the CPU 111 of the handheld device 110 reads the program stored in the determination result transmitting unit provided in the ROM 117 into the RAM 118, and transmits the result of being determined to satisfy the criterion to the server device 140. The server device 140 advances, based on the result of this determination, the processing to step S706 (FIG. 7) as will be mentioned later on. Furthermore, the determination result transmitting unit may be enabled to execute a process of further transmitting, simultaneously with the result of the determination, the data record (FIG. 2A) indicating the user-specific information stored in the user-specific information storage unit 125 to the server device 140. This mode being adopted, only if the user's biometric information satisfies the predetermined criterion, the user-specific information is transmitted to the server device 140, which therefore leads to a decrease in processing load of the server device.

The processing result of the user's biometric information in the server device 140 or the handheld device 110 can be, as will be described later on with reference to the flowchart in FIG. 7, used as a criterion for determining whether or not the server device 140 transmits the user-specific information to the necessary medical facility terminal 170.

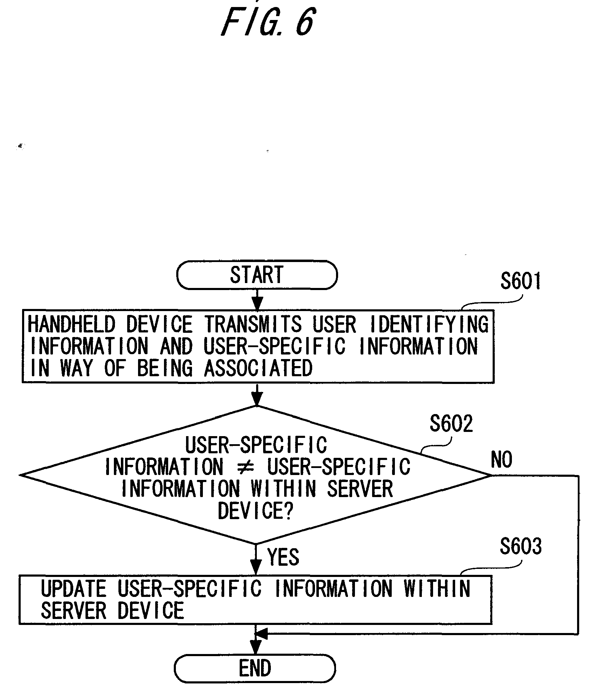

Next, a method by which the server device 140 updates the user-specific information stored in the user-specific information storage unit 150 within this server device 140, will be described with reference to a flowchart in FIG. 6.

The CPU 111 of the handheld device 110 transmits the data record (FIG. 2A) stored in the user-specific information storage unit 125 to the server device 140. This transmitting operation may take a mode that the user arbitrarily instructs the CPU 111 to transmit the data record via the input unit 123 and may also take a mode that the CPU 111 periodically automatically transmits the data record. Alternatively, there may be taken still another mode that the user inputs the latest user-specific information to the handheld device 110, and the CPU 111 automatically transmits this user-specific information to the server device 140 simultaneously with updating the user-specific information storage unit 125 (step S601).

Next, the CPU 141 of the server device 140 receives the data record (FIG. 2A) transmitted from the handheld device 110 and stores this data record in the RAM 142. Subsequently, the CPU 141 reads the program stored in the user-specific information updating unit 154 existing within the ROM 147 into the RAM 142 and executes the next process. At first, the CPU 141 refers to the user-specific information storage unit 150 in the server device 140 and compares the user-specific information of the data record (FIG. 2C) in the user-specific information storage unit 150 of the server device 140 with the user-specific information of the data record (FIG. 2A) transmitted from the handheld device 110 (step S602). The CPU 141, if different (S602; YES), advances the processing to step S603. Whereas if coincident (S602; NO), the CPU 141 erases the program read from the user-specific information updating unit 154, thus terminating the processing.

Subsequently, in step S603, the CPU 141 updates the user-specific information of the data record (FIG. 2C) in the user-specific information storage unit 150 of the server device 140 into the user-specific information of the data record (FIG. 2A) transmitted from the handheld device 110 (step S603). After updating, the CPU 141 terminates the processing by erasing the data record (FIG. 2A) read into the RAM 142 and the program read from the user-specific information updating unit 154.

The thus-made transmission and reception of the information between the handheld device 110 and the server device 140 enable the user-specific information held by the user to be kept in the latest status.

Next, a method by which the server device 140 transmits the user-specific information to the necessary medical facility terminal 170 will be described with reference to a flowchart in FIG. 7. Note that FIG. 7 illustrates respective steps in the way of being associated with items of data stored in the RAM 142 of the server device 140 when executing these steps of a processing flow.

The CPU 111 of the handheld device 110 periodically transmits, by way of signals, the pieces of user identifying information stored in the user identifying information storage unit 124. All of the access points 130 capable of receiving these signals transmit the signals to the server device 140 in a way that attaches pieces of access point information allocated to the individual access points and the reception intensities to the signals (step S701).

Next, the CPU 141 of the server device 140 receives the user identifying information, the access point information and the reception intensity that are transmitted via the access point 130 in step S701, and stores these items of information in the RAM 142. Subsequently, the CPU 141 reads the program stored in the access point information determining unit 155 existing in the ROM 147 into the RAM 142, and executes the following processes.

To start with, the CPU 141 compares the reception intensities transmitted from the individual access points with each other in step S701. The CPU 141 selects the access point information received at the strongest reception intensity and stores this access point information in the way of being associated with the user identifying information in the RAM 142. The CPU 141 erases, other than the thus-stored access point information, the remaining pieces of access point information and the reception intensity information as well. Through this process, the server device 140 acquires the access point information indicating the access point to which the handheld device 110 belongs at the present (step S702).

Next, the CPU 141 refers to the database (FIG. 2B) of the access point information storage unit 152 that is stored in the auxiliary storage unit 148, and searches for the user identifying information coincident with the user identifying information stored in step S702, thereby specifying the access point information associated with this user identifying information. Then, the CPU 141 compares this specified information with the access point information stored in the RAM 142. The CPU 141, if different, determines that there changes the access point to which the handheld device 110 belongs (S703; YES), and advances the processing to step S704. The CPU 141, if coincident, determines that the handheld device 110 remains belonging to the same access point (S703; NO). In this case, the CPU 141 erases the user identifying information and the access point information which are stored in the RAM 142 and the program read from the access point information determining unit 155, thereby finishing the processing.

Subsequently, the CPU 141 carries out the process of updating the access point information specified on the database (FIG. 2B) of the access point information storage unit 152 that is stored in the auxiliary storage unit 148 into the access point information stored in the RAM 142 (step S704). After updating, the CPU 141 erases the program read from the access point information determining unit 155 that is stored in the RAM 142 but makes the access point information and the user identifying information each stored in the RAM 142 remain as they are.

The CPU 141, after erasing the program read from the access point information determining unit 155 that is stored in the RAM 142 in step S704, reads next the program stored in the biometric information prediction determining unit 158 provided in the ROM 147 into the RAM 142. With this operation, the CPU 141 executes the process of determining, as described above in FIG. 4, whether the blood glucose level satisfies the predetermined criterion or not (step S705). When determining that the blood glucose level satisfies the predetermined criterion (S705; YES), the CPU 141 erases only the program read from the biometric information prediction determining unit 158 that is stored in the RAM 142 but makes the access point information and the user identifying information each stored in the RAM 142 remain as they are, and advances the processing to step S706. While on the other hand, when determining that the predetermined criterion is not satisfied (S705; NO), the CPU 141 terminates the processing by erasing the access point information and the user identifying information each stored in the RAM 142 and the program read from the biometric information prediction determining unit 158.

The CPU 141, after erasing only the program read from the biometric information prediction determining unit 158 that is stored in the RAM 142 in step S705, reads next the program stored in the medical facility terminal specifying unit 161 existing in the ROM 147 into the RAM 142. With this operation, the CPU 141 executes the process of specifying the medical facility terminal 170 located within the communication-enabled region with the access point to which the handheld device 110 belongs and transmitting the user-specific information to this specified medical facility terminal 170.

To be specific, at first, the CPU 141 refers to the database (FIG. 2D) of the medical facility terminal storage unit 151 that is stored in the auxiliary storage unit 148 and searches for the access point information coincident with the access point information stored in the RAM 142 from within this database, thus specifying all pieces of medical facility terminal identifying information associated with this access point information (step S706). Next, the CPU 141 executes the process of again storing the user identifying information stored in the RAM 142 and the specified medical facility terminal identifying information in the way of being associated with each other, and, simultaneously with this storing process, erasing the access point information stored in the RAM 142.

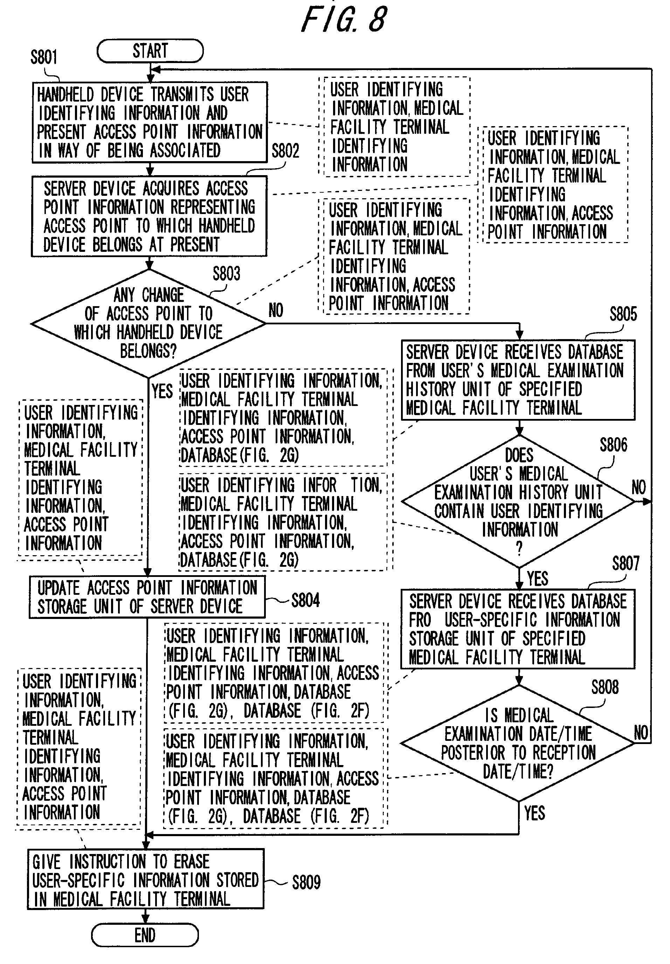

Subsequently, the CPU 141 refers to the database (FIG. 2C) of the user-specific information storage unit 150 that is stored in the auxiliary storage unit 148, and searches for the user-specific information coincident with the user-specific information stored in the RAM 142 from within this database, thereby specifying the user-specific information associated with this user identifying information. Then, the CPU 141 performs the process of further associating this specified user-specific information with the user identifying information and the medical facility terminal identifying information that are stored in the RAM 142 and again storing these associated items of information in the RAM 142. Next, the CPU 141 executes the process of transmitting these items of information in the way of being associated with each other in the RAM 142 to the medical facility terminal 170 specified by the medical facility terminal identifying information in step S706 (step S707). Note that the transmission may take a unidirectional transmission mode to the medical facility terminal 170 from the server device 140 or may also take a bidirectional transmission mode to the specified medical facility terminal 170 from the server device 140 after the CPU 171 of the medical facility terminal 170 has instructed the CPU 141 of the server device 140 to send the user-specific information to the medical facility terminal 170 via the transmission/reception unit 174. After the transmission, the CPU 141 erases the user-specific information and the program read from the medical facility terminal specifying unit 161 that are stored in the RAM 142, and finishes the processing. Note that the user identifying information and the medical facility terminal identifying information associated therewith are used in the processes from step S801 onward that will be explained in FIG. 8 and are therefore consecutively stored in the RAM 142 (step S708).

Further, after step S707, on the medical facility terminal 170 receiving the user-specific information, the CPU 171 of the medical facility terminal 170 stores the received items of information, i.e., the user-specific information and the user identifying information in the RAM 172, and simultaneously acquires the received date/time information (reception date/time) from the clock 178. Then, the received user-specific information, the received user identifying information and the acquired date/time information are stored in the way of being associated with each other by way of a database in the user-specific information storage unit 185 of the auxiliary storage unit 177 (FIG. 2F). After storing the information, the CPU 171 erases the user identifying information and the user-specific information each stored in the RAM 172, and finishes the processing.

The user-specific information stored in the user-specific information storage unit 185 of the medical facility terminal 170 is checked by a medical worker who inputs, when the user undergoes the medical examination in the medical facility or carried to the medical facility, the user identifying information of the handheld device 110 held by the user to the input unit 179. After inputting, the user-specific information associated with this user identifying information in the database stored in the user-specific information storage unit 185 is displayed on the display unit 180 and can be thus checked. Note that upon inputting the user identifying information to the input unit 179, the inputted date/time information (medical examination date/time) is acquired from the clock 178, and the user identifying information and the medical examination date/time are stored in the way of being associated with each other as the database in the user's medical examination history unit 184 of the auxiliary storage unit 177 (FIG. 2G). Incidentally, there may be provided a configuration enabling the medical worker to check the user-specific information by accessing beforehand the user-specific information storage unit 185 before the user undergoes the medical examination in the medical facility or carried to the medical facility.

Incidentally, such an available scheme may also be taken that the CPU 111 of the handheld device 110 transmits the user identifying information in step S701, but instead the CPU 141 of the server device 140 periodically transmits the user identifying information via all of the access points 130 and determines which access point the handheld device 110 associated with the user identifying information receives the information with the strongest reception intensity at. In this case, in step S702, the transmitted user identifying information and the access point information of the access point at which to receive the information with the strongest reception intensity are stored in the RAM 142 of the server device 140.

Note that step S705 may be inserted in between the start (Start) of the processing of the server device 140 and the step S701, and, only when satisfying the predetermined criterion in step S705, the process in step S701 may be advanced. If contrived in this way, when determining that the blood glucose level of the user does not satisfy the predetermined criterion, there is no necessity for the subsequent processes by the server device 140, and it is therefore feasible to reduce the loads applied on the processes of the handheld device 110 and on the processes of the server device 140.

Incidentally, as for step S706 and step S707, other than the medical facility terminal 170 in the communication-enabled region with the access point to which the handheld device 110 belongs, the access point existing in the region neighboring to the belonging access point is also specified, and the user-specific information may be sent to the medical facility terminal in the communication-enabled region with this access point. If thus contrived, if the user employing the handheld device 110 makes a speedy movement by column or by car, and, even when the belonging access point abruptly changes, the proper and prompt treatment can be implemented for the user in the medical facility because of the user-specific information being sent beforehand to the medical facility terminal existing in the communication-enabled region with the access point to which the handheld device 110 reaching the destination of the movement belongs.

Note that if the handheld device 110 is immobilized within the belonging access point or if the user employing the handheld device 110 hardly ever exits the communication-enabled region with the belonging access point, the processes in step S701 through step S704 may be omitted, and only the processes from step S705 onward may be executed. With this contrivance, without user's being aware of the fluctuations of his or her own blood glucose level and when the predetermined criterion is satisfied, the server device 140 automatically transmits the user-specific information to the medical facility terminal 170, and hence, even if the user is carried to the medical facility but unable to conduct the mutual communications with the medical worker, a proper medical care or a proper diagnosis or a proper dosage of medicine is carried out.

It is to be noted that the user using the handheld device 110 swiftly moves across the plurality of access points 130 in a short period of time, it is determined in step S705 that the predetermined criterion is satisfied, and the user-specific information is sent to the medical facility terminal 170, on which occasion if the user has already moved to another access point, step S705 is omitted, and only the processes in remaining steps S701 - S704 and S706 to S707 may be executed. With this contrivance, immediately when the handheld device 110 moves to a different access point, the server device 140 can send the user-specific information to the medical facility terminal 170 located in the communication-enabled region with the access point to which the handheld device 110 has moved, and therefore, even if the user is carried to the medical facility but unable to conduct the mutual communications with the medical worker, the proper medical care or the proper diagnosis or the proper dosage of medicine is implemented.

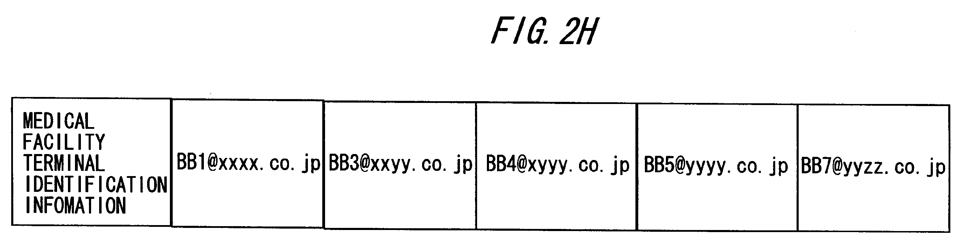

Note that there is a case where the user-specific information does not need sending to all of the medical facility terminals 170 located within the communication-enabled region with a certain access point but is sent to only the medical facility terminal 170 specified by the user. In this case, the auxiliary storage unit 116 of the handheld device 110 is newly provided with a medical facility terminal specifying unit (unillustrated in FIG. 1B), and the handheld device 110 stores the medical facility terminal identifying information associated with the medical facility terminal 170 specified beforehand by the user in the form of the database shown in, e.g., FIG. 2H in the medical facility terminal specifying unit. Then, in step S701, the CPU 111 of the handheld device 110 sends all pieces of the medical facility terminal identifying information stored in the medical facility terminal specifying unit in addition to the user identifying information in the way of being associated with each other to the CPU 141 of the server device 140, and stores the medical facility terminal identifying information together with the user identifying information in the RAM 142 of the server device 140 till the process in step S705.

Next, in step S706, the CPU 141 reads the program stored in the medical facility terminal specifying unit 161 existing in the ROM 147 into the RAM 142, then refers to the database (FIG. 2D) of the medical facility terminal storage unit 151 that is stored in the auxiliary storage unit 148, and specifies the medical facility terminal identifying information associated with the access point information coincident with the access point information stored in the RAM 142 in this database. Then, the CPU 141 determines whether or not this specified medical facility terminal identifying information is coincident with the medical facility terminal identifying information derived from the medical facility terminal specifying unit that is simultaneously stored in the RAM 142, and erases only the discrepant medical facility terminal identifying information. Then, the CPU 141 uses the remaining pieces of medical facility terminal identifying information in the RAM 142 for the process in step S707. This contrivance enables the user-specific information to be sent to only the medical facility terminal specified previously by the user. If it is known beforehand that the user grasps the medical facility where the user is subjected to the optimal treatment or grasps the well-accustomed medical facility and undergoes the medical examination in that medical facility, the process of providing the medical facility terminal specifying unit may also be executed.

Owing to the transmission and the reception of the information among the handheld device 110, the server device 140 and the medical facility terminal 170, the user has no necessity for performing the predetermined operations separately even when the handheld device 110 belongs to the new access point or the blood glucose level satisfies the predetermined criterion, and the user-specific information is previously sent to all of the medical facility terminals 170 within the communication-enabled region with the access point to which the handheld device 110 belongs. Then, owing to the transmission and the reception of the information such as this, even if the user falls into the comatose state due to, e.g., the hypoglycaemia and reaches a state of being disabled from making the mutual communications with the medical facility, the medical facility can grasp the pre-sent user-specific information and can implement the proper medical care or the proper diagnosis or the proper dosage of medicine for the user.

Next, after the user-specific information has been sent to the medical facility terminal 170 in the communication-enabled region with the belonging access point, if the access point to which the handheld device 110 of this user belongs is changed, or if the user has already undergone the medical examination in the medical facility or already been carried to the medical facility in which to install the medical facility terminal 170 to which the user-specific information is sent, the medical facility terminal 170 has no necessity for continuing to store the user-specific information existing in the user-specific information storage unit 185.

Herein, an operation of how the server device 140 erases the user-specific information stored in the user-specific information storage unit 185 in the medical facility terminal 170, will be described with reference to a flowchart in FIG. 8. Note that FIG. 8 shows respective steps in the way of being associated with items of data stored in the RAM 142 of the server device 140 when executing these steps of a processing flow.

The CPU 111 of the handheld device 110 periodically transmits, by way of signals, the pieces of user identifying information stored in the user identifying information storage unit 124. All of the access points capable of receiving these signals transmit the signals to the server device 140 in a way that attaches the access point information allocated to the individual access points and the reception intensities to the signals (step S801).

Next, the CPU 141 of the server device 140 receives the user identifying information coincident with the user identifying information continuously stored in the RAM 142 from step S707 onward in FIG. 7, the access point information and the reception intensity that are transmitted via the access point in step S801, and stores these items of information in the RAM 142. Subsequently, the CPU 141 reads the program stored in the access point information determining unit 155 existing in the ROM 147 into the RAM 142, and executes the following processes. To start with, the CPU 141 compares the reception intensities transmitted from the individual access points with each other in step S801. Then, the CPU 141 selects the access point information received at the strongest reception intensity and stores this access point information in the RAM 142. The CPU 141 erases, other than the thus-stored access point information, the remaining pieces of access point information and the reception intensity information as well. Through this process, the server device 140 acquires the access point information indicating the access point to which the handheld device 110 belongs at the present (step S802).

Next, the CPU 141 refers to the database (FIG. 2B) of the access point information storage unit 152 that is stored in the auxiliary storage unit 148, and searches for the user identifying information coincident with the user identifying information continuing to be stored in the RAM 142 from step S707 onward in FIG. 7, thereby specifying the access point information associated with this user identifying information. Subsequently, the CPU 141 compares this specified information with the access point information stored in the RAM 142. The CPU 141, if different, after the server device 140 has sent the user-specific information to the medical facility terminal 170, determines that there changes the access point to which the handheld device 110 belongs (S803; YES), and advances the processing to step S804. The CPU 141, if coincident, determines that the handheld device 110 remains belonging to the same access point (S803; NO), then erases the program read from the access point information determining unit 155, and thereafter advances the processing to step S805.

Subsequently, the CPU 141 carries out the process of updating the access point information specified in step S803 on the database (FIG. 2B) of the access point information storage unit 152 that is stored in the auxiliary storage unit 148 into the access point information stored in the RAM 142 (step S804). After updating, the CPU 141 erases the program read from the access point information determining unit 155 that is stored in the RAM 142. Note that the user identifying information and the medical facility terminal identifying information associated with this user identifying information, which have been stored in the RAM 142 from step S707 onward in FIG. 7, remain stored therein.

While on the other hand, when determining in step S803 that the handheld device 110 remains belonging to the same access point (S803; NO), the CPU 141 of the server device 140 executes a process of determining whether or not the user employing the handheld device 110 undergoes the medical examination with respect to the medical facility terminal 170 located within the communication-enabled region with this access point. In other words, the CPU 141 executes the process of determining whether or not the user has already undergone the medical examination in the medical facility in which this medical facility terminal 170 is installed or already been carried to this medical facility. A series of these processes will be explained in steps S805, S806, S807 and S808 that will be given as below.

Next, the CPU 141 of the server device 140 reads the program stored in the user's medical examination history checking unit 159 existing in the ROM 147 into the RAM 142, and executes the following processes. To begin with, the CPU 141 of the server device 140 instructs the CPU 171 of the medical facility terminal 170 specified based on the medical facility terminal identifying information continuing to be stored in the RAM 142 from step S707 onward in FIG. 7 to transmit the database (FIG. 2G) of the user's medical examination history unit 184 that is stored in the auxiliary storage unit 177 to the server device 140. Then, the CPU 141 carries out the process of storing the database (FIG. 2G) transmitted from the medical facility terminal 170 in the RAM 142 (step S805).

Subsequently, the CPU 141 refers to this database and searches for the user identifying information coincident with the user identifying information continuing to be stored in the RAM 142 from step S707 onward in FIG. 7, thus determining whether there is the coincident user identifying information or not (step S806). If the coincident user identifying information is not recognized (S806; NO), the CPU 141 erases this database and the access point information that are stored in the RAM 142 and the program read from the user's medical examination history checking unit 159, and returns to the start in FIG. 8. Then, the CPU 141 consecutively waits for the handheld device 110 to transmit the user identifying information in step S801. Whereas if the coincident user identifying information is recognized (S806; YES), the CPU 141 advances the processing to step S807 while keeping this database stored in the RAM 142.

Next, the CPU 141 instructs the CPU 171 of the medical facility terminal 170 specified based on the medical facility terminal identifying information continuing to be stored in the RAM 142 from step S707 onward in FIG. 7 to transmit the database (FIG. 2F) of the user-specific information storage unit 185 existing in the auxiliary storage unit 177 to the server device 140. Then, the CPU 141 executes the process of storing the database sent from the medical facility terminal 170 in the RAM 142 (step S807). Note that the medical facility terminal 170 may take a mode of transmitting not the database (FIG. 2F) itself of the user-specific information storage unit 185 but a database (in which there remain items of data such as the user identifying information and the reception date/time that are associated with each other) configured by removing only the user-specific information from the database (FIG. 2F).

Subsequently, the CPU 141 searches for the user identifying information coincident with the user identifying information continuing to be stored in the RAM 142 from step S707 onward in FIG. 7 on the database stored in the RAM 142 in step S805 and the database (FIG. 2F) stored in the RAM 142 in step S807, and specifies the medical examination date/time and the reception date/time each associated with this user identifying information from the database (FIG. 2G) and the database (FIG. 2F) as well. Then, the CPU 141 compares the medical examination date/time and the reception date/time with each other. The CPU 141, if the medical examination date/time is posterior to the reception date/time (S808; YES), determines that the user undergoes the medical examination in the medical facility where the medical facility terminal 170 is installed. With this determination, the CPU 141 erases the database (FIG. 2G) and the database (FIG. 2F) each stored in the RAM 142 and the program read from the user's medical examination history checking unit 159, and advances the processing to step S809. Whereas if the medical examination date/time is not posterior to the reception date/time (S808; NO), the CPU 141, after erasing the database (FIG. 2G), the database (FIG. 2F) and the access point information each stored in the RAM 142 and the program read from the user's medical examination history checking unit 159, returns to the start in FIG. 8. Then, the CPU 141 consecutively waits for the handheld device 110 to transmit the user identifying information in step S801.

Next, the CPU 141 of the server device 140 reads the program stored in the user-specific information erasing unit 160 existing in the ROM 147 into the RAM 142, and executes the following processes. The CPU 141 instructs the CPUs 171 of all of the medical facility terminals 170 specified based on the medical facility terminal identifying information continuing to be stored in the RAM 147 from step S707 onward in FIG. 7 to erase the user identifying information coincident with the user identifying information continuing to be stored in the RAM 142 from step S707 onward in FIG. 7 and all pieces of information (the reception date/time, the user-specific information) associated with the user identifying information on the database (FIG. 2F) stored in the user-specific information storage unit 185 of the auxiliary storage unit 177. Further, the CPU 141 gives the instruction of erasing the user identifying information coincident with the user identifying information continuing to be stored in the RAM 142 of the server device 140 from step S707 onward in FIG. 7 and the medical examination date/time associated with the user identifying information similarly on the database (FIG. 2G) stored in the user's medical examination history unit 184 of the auxiliary storage unit 177. After the instruction process, the CPU 141 of the server device 140 erases the user identifying information continuing to be stored in the RAM 142, the medical facility terminal identifying information and the access point information each associated therewith and the program read from the user-specific information erasing unit 160, and finishes the processing (step S809).

Incidentally, such an available scheme may also be taken that the CPU 111 of the handheld device 110 transmits the user identifying information in step S801, but instead the CPU 141 of the server device 140 periodically transmits the user identifying information via all of the access points 130 and determines which access point the handheld device 110 associated with the user identifying information receives the information with the strongest reception intensity at. In this case, the CPU 141, in step S802, stores the transmitted user identifying information and the access point at which to receive the information with the strongest reception intensity in the RAM 142 of the server device 140.

With the transmission and the reception of the information among the handheld device 110, the server device 140 and the medical facility terminal 170, the user-specific information having no necessity of continuing to be stored on the medical facility terminal 170 does not separately require the predetermined operations on the side of the medical facility and can be automatically erased by the instruction given from the server device 140. Then, owing to the transmission and the reception of the information such as this, the user-specific information is stored only on the medical facility terminal 170 belonging to the communication-enabled region with the access point within a period for which the user remains belonging to this access point and is therefore, it follows, dealt with within the minimum range outside the handheld device 110. As a result, the user-specific information can be prevented from leaking outside the medical facility.

The present embodiment does not restrict a technique of realizing the respective processing units such as the access point information determining unit 155, which are included by the handheld device 110, the server device 140 and the medical facility terminal 170. The respective processing units may be configured as hardware components or software components or combinations thereof by a technique realizable to ordinary engineers in the field of the present technology.

The hardware component is a hardware circuit which can be exemplified by an FPGA (Field Programmable Gate Array), an ASIC (Application Specific Integrated Circuit), a gate array, a combination of logic gates, a signal processing circuit, an analog circuit, etc. The software component is defined as a component (fragment) which realizes softwarewise the process described above but is not a concept which limits a language, a development environment, etc for realizing the software. The software component is exemplified by, e.g., a task, a thread, a driver, firmware, a database, a table, a function, a procedure, a subroutine, a predetermined segment of a program code, a data structure, an array, a variable and a parameter. These software components are realized on a single memory or a plurality of memories in the computer or realized by a single processor or a plurality of processors (e.g., a CPU (Central Processing Unit), DSP (Digital Signal Processor), etc) which run the data on the single or plural memories.

Industrial Applicability

According to the present invention, even if the user falls into the comatose state due to, e.g., the hypoglycaemia and reaches the state of being disabled from making the mutual communications with the medical facility, the medical facility in which to install the medical facility terminal can grasp the pre-sent user-specific information and can implement the proper medical care or the proper diagnosis or the proper dosage of medicine for the user. On the other hand, if the sent user-specific information becomes unnecessary, the predetermined operations are not performed separately on the side of the medical facility terminal, but the automatically erasing process is executed on the side of the server device, and hence the user-specific information can be safely handled without any necessity for taking account of the working load for separately managing the user-specific information. As a result, it is feasible to save the time-consuming operations of managing the user-specific information, which are burdened onto both of the user and the medical facility.

Description of the Reference Numerals and Symbols

110 handheld device
111 CPU (of handheld device)
112 measuring unit
113 analog signal processing unit
114 analog-to-digital (A/D) conversion unit
115 transmission/reception unit (of handheld device)
116 auxiliary storage unit (of handheld device)
117 ROM (of handheld device)
118 RAM (of handheld device)
119 EEPROM (of handheld device)
120 clock (of handheld device)
121 gate array (of handheld device)

122 display unit (of handheld device)
123 input unit (of handheld device)
124 user identifying information storage unit
125 user-specific information storage unit (of handheld device)
126 measurement data storage unit
127 calibration curve data storage unit
128 modem (of handheld device)
129 NCU (of handheld device)
130 access point
131 1-133 access point installed in each region
134 communication network
140 server device
141 CPU 141 (of server device)
142 RAM (of server device)
143 NCU (of server device)
144 modem (of server device)
145 transmission/reception unit (of server device)
146 EEPROM (of server device)
147 ROM (of server device)
148 auxiliary storage unit (of server device)
149 clock (of server device)
150 user-specific information storage unit (of server device)
151 medical facility terminal storage unit
152 access point information storage unit
153 profile generation storage unit
154 user-specific information updating unit
155 access point information determining unit
156 biometric information profile generation unit
157 fluctuation pattern specifying unit
158 biometric information prediction determining unit
159 user's medical examination history checking unit
160 user-specific information erasing unit
161 medical facility terminal specifying unit

## Claims

1. A user-specific information provision system (100) comprising a handheld device (110), a server device (140) and a medical facility terminal (170);
the handheld device (110) configured to transmit, to the server device (140), user-specific information held specifically by a user and user's biometric information acquired by measuring biometric information of the user;
the server device (140) configured to receive the user-specific information and the user's biometric information; and
the medical facility terminal (170) configured to receive information transmitted from the server device (140),
the server device (140) including an access point information determining unit (155) configured to acquire access point information representing an access point (130) to which the handheld device (110) belongs at present and to determine whether the access point (130) is changed or not,
at least one of the handheld device (110) and the server device (140) further including a biometric information prediction determining unit configured to determine whether or not the user's biometric information satisfies a predetermined criterion,
**characterised in that**
the server device (140) is configured to transmit the user-specific information to the medical facility terminal (170) when both the access point information determining unit (155) determines that the access point is changed and the biometric information prediction determining unit determines that the predetermined criterion is satisfied.

2. The user-specific information provision system according to claim 1, wherein the server device (140) further includes an access point information storage unit (152) configured to store access point information of the handheld device (110), and
the access point information determining unit (155) is configured to determine that the access point (130) to which the handheld device (110) belongs is changed when the acquired access point information is different from the access point information stored in the access point information storage unit (152).

**3.** The user-specific information provision system according to claim 1 or 2, wherein at least one of the handheld device (110) and the server device (140) further includes:

a biometric information profile generation unit (156) configured to generate a user-specific biometric information profile, based on past user's biometric information of the user, with which a fluctuation pattern of the past user's biometric information and a fluctuation value of prospective user's biometric information when the fluctuation pattern occurs are associated; and

a fluctuation pattern specifying unit (157) configured to specify the fluctuation pattern of the past user's biometric information, which is coincident with a fluctuation pattern of recent user's biometric information of the user, among the biometric information profile generated by the biometric information profile generation unit (156), and the biometric information prediction determining unit (158) is further configured to calculate a predictive value of the prospective user's biometric information by adding the fluctuation value of the prospective user's biometric information, which is associated with the fluctuation pattern of the past user's biometric information specified by the fluctuation pattern specifying unit (157), to present user's biometric information, and is further configured to determine whether the user's biometric information satisfies the predetermined criterion or not by using the predictive value of the prospective user's biometric information.

**4.** The user-specific information provision system according to claim 1, wherein the medical facility terminal (170) further includes a user-specific information storage unit (185) configured to store the transmitted user-specific information, and

the server device (140) is configured to erase the user-specific information stored in the user-specific information storage unit (185) of the medical facility terminal (170) when the access point information determining unit (155) determines that the access point (130) to which the handheld device (110) belongs is changed after the server device (140) has transmitted the user-specific information to the medical facility terminal (170).

**5.** A user-specific information provision method executed by a server device (140) capable of receiving, from a handheld device (110), user-specific information held specifically by a user and user's biometric information acquired by measuring biometric information of the user, the server device (140) receiving the user-specific information and the user's biometric information, the server device (140) being capable of transmitting information to a medical facility terminal (170), the method comprising:

a step (S702) of the server device (140) acquiring access point information representing an access point (130) to which the handheld device (110) belongs at present and determining whether the access point (130) is changed or not;

a biometric information prediction determining step (S705) of the server device (140) determining whether the user's biometric information satisfies a predetermined criterion or not, or the server device (140) receiving from the handheld device (110) the result of a determination made by the handheld device (110) as to whether the user's biometric information satisfies a predetermined criterion or not;

an access point information determining step (S703) of the server device (140) determining whether the access point (130) is changed or not; and

a step (S707) of the server device (140) transmitting the user-specific information to the medical facility terminal (170) when it is determined that both the predetermined criterion is satisfied and the access point (130) is changed.

**6.** The user-specific information provision method according to claim 5, wherein the server device (140) further includes an access point information storage unit (152) to store access point information of the handheld device (110), and the access point information determining step determines that the access point (130) to which the handheld device (110) belongs is changed when the acquired access point information is different from the access point information stored in the access point information storage unit (152).

**7.** The user-specific information provision method according to claim 5, wherein the biometric information prediction determining step further includes:

a biometric information profile generation step of generating a user-specific biometric information profile, based on past user's biometric information of the user, with which a fluctuation pattern of the past user's biometric information and a fluctuation value of prospective user's biometric information when the fluctuation pattern occurs are associated;

a fluctuation pattern specifying step of specifying the fluctuation pattern of the past user's biometric information, which is coincident with a fluctuation pattern of recent user's biometric information of the user, among the biometric information profile generated in the biometric information profile generation step;

a step of calculating a predictive value of the prospective user's biometric information by adding the fluctuation value of the prospective user's biometric information when the fluctuation pattern of the past user's biometric information occurs, which is specified in the fluctuation pattern specifying step, to present user's biometric information; and

a step of determining by using the predictive value of the prospective user's biometric information whether the user's biometric information satisfies the predetermined criterion or not.

8. The user-specific information provision method according to claim 5, wherein the medical facility terminal (170) further includes a user-specific information storage unit (185) to store the transmitted user-specific information, and the method comprises:

an access point information determining step (S803) of the server device (140) determining that the access point (130) to which the handheld device (110) belongs is changed after the step of transmitting the user-specific information to the medical facility terminal (170); and

a user-specific information erasing step (S809) of the server device (140) erasing the user-specific information stored in the user-specific information storage unit (185) of the medical facility terminal (170) when determining that the access point (130) is changed.

9. A server device (140) configured to receive user-specific information transmitted from a handheld device (110) and held specifically by a user and user's biometric information acquired by measuring biometric information of the user, the server device (140) comprising:

an access point information determining unit (155) configured to acquire access point information representing an access point (130) to which the handheld device (110) belongs at present and to determine whether the access point is changed or not;

a biometric information prediction determining unit (158) configured to determine whether or not the user's biometric information satisfies a predetermined criterion; and

**characterised by**

a medical facility terminal specifying unit (161) configured to transmit the user-specific information to a medical facility terminal (170) when it is determined that both the access point is changed and the predetermined criterion is satisfied.

10. The server device according to claim 9, further comprising an access point information storage unit (152) configured to store access point information of the handheld device (110),

wherein the access point information determining unit (155) is configured to determine that the access point (130) to which the handheld device (110) belongs is changed when the acquired access point information is different from the access point information stored in the access point information storage unit (152).

11. The server device according to claim 9, further comprising:

a biometric information profile generation unit (156) configured to generate a user-specific biometric information profile, based on past user's biometric information of the user, with which a fluctuation pattern of the past user's biometric information and a fluctuation value of prospective user's biometric information when the fluctuation pattern occurs are associated; and

a fluctuation pattern specifying unit (157) configured to specify the fluctuation pattern of the past user's biometric information, which is coincident with a fluctuation pattern of user's recent biometric information of the user, from within the biometric information profile generated by the biometric information profile generation unit (156),

wherein the biometric information prediction determining unit (158) is further configured to calculate a predictive value of the prospective user's biometric information by adding the fluctuation value of the prospective user's biometric information that is associated with the fluctuation pattern of the past user's biometric information specified by the fluctuation pattern specifying unit (157), to present user's biometric information, and is further configured to determine by using the predictive value of the prospective user's biometric information whether the user's biometric information satisfies the predetermined criterion or not.

12. The server device according to claim 9, further comprising a user-specific information erasing unit (160) configured to erase the user-specific information stored on the medical facility terminal (170), when the access point information determining unit (155) determines that the access point (130) to which the handheld device (110) belongs is changed after transmitting the user-specific information to the medical facility terminal (170).

**Patentansprüche**

1. Benutzerspezifisches Informationsbereitstellungssystem (100), das Folgendes umfasst: eine Handvorrichtung (110), eine Servervorrichtung (140) und ein Endgerät (170) einer medizinischen Vorrichtung;
   wobei die Handvorrichtung (110) dafür konfiguriert ist, zu der Servervorrichtung (140) Folgendes zu übertragen: benutzerspezifische Informationen, die ein Benutzer spezifisch speichert, und biometrische Benutzerinformationen, die durch Messung der biometrischen Informationen des Benutzers erfasst werden;
   wobei die Servervorrichtung (140) dafür konfiguriert ist, die benutzerspezifischen Informationen und die biometrischen Benutzerinformationen zu empfangen; und
   wobei das Endgerät (170) der medizinischen Vorrichtung dafür konfiguriert ist, Informationen zu empfangen, die von der Servervorrichtung (140) übertragen werden,
   wobei die Servervorrichtung (140) eine Zugriffspunkt-Informationsbestimmungseinheit (155) umfasst, die für Folgendes konfiguriert ist: Erhalten von Zugriffspunktinformationen, die einen Zugriffspunkt (130) darstellen, zu dem die Handvorrichtung (110) gegenwärtig gehört, und Bestimmen, ob der Zugriffspunkt (130) geändert ist oder nicht, wobei mindestens die Handvorrichtung (110) oder die Servervorrichtung (140) ferner eine Prognosebestimmungseinheit für biometrische Informationen umfasst, die dafür konfiguriert ist, zu bestimmen, ob die biometrischen Benutzerinformationen ein vorbestimmtes Kriterium erfüllen oder nicht,
   **dadurch gekennzeichnet, dass** die Servervorrichtung (140) dafür konfiguriert ist, die benutzerspezifischen Informationen zu dem Endgerät (170) der medizinischen Vorrichtung zu übertragen, wenn sowohl die Zugriffspunkt-Informationsbestimmungseinheit (155) bestimmt, dass der Zugriffspunkt geändert ist, als auch die Prognosebestimmungseinheit für biometrische Informationen bestimmt, dass das vorbestimmte Kriterium erfüllt ist.

2. Benutzerspezifisches Informationsbereitstellungssystem nach Anspruch 1, wobei die Servervorrichtung (140) ferner Folgendes umfasst: eine Zugriffspunkt-Informationsspeichereinheit (152), die dafür konfiguriert ist, Zugriffspunktinformationen der Handvorrichtung (110) zu speichern und
   wobei die Zugriffspunkt-Informationsbestimmungseinheit (155) dafür konfiguriert ist, zu bestimmen, dass der Zugriffspunkt (130), zu dem die Handvorrichtung (110) gehört, geändert ist, wenn sich die erworbenen Zugriffspunktinformationen von den Zugriffspunktinformationen, die in der Zugriffspunkt-Informationsspeichereinheit (152) gespeichert sind, unterscheiden.

3. Benutzerspezifisches Informationsbereitstellungssystem nach Anspruch 1 oder 2, wobei mindestens die Handvorrichtung (110) oder die Servervorrichtung (140) ferner Folgendes umfasst:

   eine Erzeugungseinheit (156) für biometrische Informationsprofile, die dafür konfiguriert ist, ein benutzerspezifisches biometrisches Informationsprofil zu erzeugen, das auf vergangenen biometrischen Benutzerinformationen des Benutzers basiert, denen ein Fluktuationsmuster der vergangenen biometrischen Benutzerinformationen und ein Fluktuationswert von zukünftigen biometrischen Benutzerinformationen zugeordnet wird, wenn das Fluktuationsmuster auftritt; und
   eine Fluktuationsmusterspezifizierungseinheit (157), die dafür konfiguriert ist, das Fluktuationsmuster der vergangenen biometrischen Benutzerinformationen zu spezifizieren, das mit einem Fluktuationsmuster der jüngsten biometrischen Benutzerinformationen des Benutzers des biometrischen Informationsprofils übereinstimmt, das von der biometrischen Informationsprofilerzeugungseinheit (156) erzeugt wird, und
   wobei die Prognosebestimmungseinheit (158) für biometrische Informationen ferner dafür konfiguriert ist, einen Prognosewert der zukünftigen biometrischen Benutzerinformationen zu berechnen, indem der Fluktuationswert der zukünftigen biometrischen Benutzerinformationen hinzugefügt wird, der dem Fluktuationsmuster der vergangenen biometrischen Benutzerinformationen zugeordnet ist, das durch die Fluktuationsmusterspezifizierungseinheit (157) spezifiziert wird, um biometrische Benutzerinformationen darzustellen und ferner dafür konfiguriert ist, zu bestimmen, ob die biometrischen Benutzerinformationen das vorbestimmte Kriterium erfüllen oder nicht, indem der Prognosewert der zukünftigen biometrischen Benutzerinformationen verwendet wird.

**4.** Benutzerspezifisches Informationsbereitstellungssystem nach Anspruch 1, wobei das Endgerät (170) der medizinischen Vorrichtung ferner eine benutzerspezifische Informationsspeichereinheit (185) umfasst, die dafür konfiguriert ist, die übertragenen benutzerspezifischen Informationen zu speichern, und
wobei die Servervorrichtung (140) dafür konfiguriert ist, die benutzerspezifischen Informationen zu löschen, die in der benutzerspezifischen Informationsspeichereinheit (185) des Endgeräts (170) der medizinischen Vorrichtung gespeichert sind, wenn die Zugriffspunkt-Informationsbestimmungseinheit (155) bestimmt, dass der Zugriffspunkt (130), zu dem die Handvorrichtung (110) gehört, geändert ist, nachdem die Servervorrichtung (140) die benutzerspezifischen Informationen zu dem Endgerät (170) der medizinischen Vorrichtung übertragen hat.

**5.** Benutzerspezifisches Informationsbereitstellungsverfahren, das von einer Servervorrichtung (140) ausgeführt wird, die in der Lage ist, von einer Handvorrichtung (110) Folgendes zu empfangen:

benutzerspezifische Informationen, die ein Benutzer spezifisch speichert, und biometrische Benutzerinformationen, die durch Messung der biometrischen Informationen des Benutzers erfasst werden, wobei die Servervorrichtung (140) die benutzerspezifischen Informationen und die biometrischen Benutzerinformationen empfängt, wobei die Servervorrichtung (140) in der Lage ist, Informationen zu einem Endgerät (170) der medizinischen Vorrichtung zu übertragen, wobei das Verfahren Folgendes umfasst:

einen Schritt (S702) des Erfassens, durch die Servervorrichtung (140), von Zugriffspunktinformationen, die einen Zugriffspunkt (130) darstellen, zu dem die Handvorrichtung (110) gegenwärtig gehört, und des Bestimmens, ob der Zugriffspunkt (130) geändert ist oder nicht,
einen Prognosebestimmungsschritt für biometrische Informationen (S705), bei dem die Servervorrichtung (140) bestimmt, ob die biometrischen Benutzerinformationen ein vorbestimmtes Kriterium erfüllen oder nicht, oder bei dem die Servervorrichtung (140) von der Handvorrichtung (110) das Ergebnis einer Bestimmung empfängt, die von der Handvorrichtung (110) durchgeführt wurde, ob die biometrischen Benutzerinformationen ein vorbestimmtes Kriterium erfüllen oder nicht;
einen Zugriffspunkt-Informationsbestimmungsschritt (S703), bei dem die Servervorrichtung (140) bestimmt, ob der Zugriffspunkt (130) geändert ist oder nicht; und
einen Schritt (S707) der Servervorrichtung (140) zum Übertragen der benutzerspezifischen Informationen zu dem Endgerät (170) der medizinischen Vorrichtung, wenn bestimmt wird, dass sowohl das vorbestimmte Kriterium erfüllt ist als auch der Zugriffspunkt (130) geändert ist.

**6.** Benutzerspezifisches Informationsbereitstellungsverfahren nach Anspruch 5, wobei die Servervorrichtung (140) ferner eine Zugriffspunkt-Informationsspeichereinheit (152) zum Speichern von Zugriffspunktinformationen der Handvorrichtung (110) umfasst und
der Zugriffspunkt-Informationsbestimmungsschritt bestimmt, dass der Zugriffspunkt (130), zu dem die Handvorrichtung (110) gehört, geändert ist, wenn sich die erworbenen Zugriffspunktinformationen von den Zugriffspunktinformationen, die in der Zugriffspunkt-Informationsspeichereinheit (152) gespeichert sind, unterscheiden.

**7.** Benutzerspezifisches Informationsbereitstellungsverfahren nach Anspruch 5, wobei der Prognosebestimmungsschritt für biometrische Informationen ferner Folgendes umfasst,
einen Erzeugungsschritt für biometrische Informationsprofile zum Erzeugen eines benutzerspezifischen biometrischen Informationsprofils, das auf vergangenen biometrischen Benutzerinformationen des Benutzers basiert, denen ein Fluktuationsmuster der vergangenen biometrischen Benutzerinformationen und ein Fluktuationswert von zukünftigen biometrischen Benutzerinformationen zugeordnet wird, wenn das Fluktuationsmuster auftritt;
einen Fluktuationsmusterspezifizierungsschritt, der das Fluktuationsmuster der vergangenen biometrischen Benutzerinformationen spezifiziert, das mit einem Fluktuationsmuster der jüngsten biometrischen Benutzerinformationen des Benutzers des biometrischen Informationsprofils übereinstimmt, das bei dem Erzeugungsschritt für biometrische Informationsprofile erzeugt wird,
einen Schritt des Berechnens eines Prognosewerts der zukünftigen biometrischen Benutzerinformationen, indem der Fluktuationswert der zukünftigen biometrischen Benutzerinformationen hinzugefügt wird, wenn das Fluktuationsmuster der vergangenen biometrischen Benutzerinformationen auftritt, das in dem Fluktuationsmusterspezifizierungsschritt spezifiziert wird, um biometrische Benutzerinformationen darzustellen; und
einen Schritt des Bestimmens unter Verwendung des Prognosewerts der zukünftigen biometrischen Benutzerinformationen, ob die biometrischen Benutzerinformationen das vorbestimmte Kriterium erfüllen oder nicht.

**8.** Benutzerspezifisches Informationsbereitstellungsverfahren nach Anspruch 5, wobei das Endgerät (170) der medizinischen Vorrichtung ferner eine benutzerspezifische Informationsspeichereinheit (185) umfasst, um die übertragenen benutzerspezifischen Informationen zu speichern, und
wobei das Verfahren Folgendes umfasst:

einen Zugriffspunkt-Informationsbestimmungsschritt (S803), bei dem die Servervorrichtung (140) bestimmt, dass der Zugriffspunkt (130), zu dem die Handvorrichtung (110) gehört, nach dem Schritt der Übertagung der benutzerspezifischen Informationen zu dem Endgerät (170) der medizinischen Vorrichtung geändert wird; und einen benutzerspezifischen Informationslöschschritt (S809), bei dem die Servervorrichtung (140) die benutzerspezifischen Informationen löscht, die in der benutzerspezifischen Informationsspeichereinheit (185) des Endgeräts (170) der medizinischen Vorrichtung gespeichert sind, wenn bestimmt wird, dass der Zugriffspunkt (130) geändert wird.

9. Servervorrichtung (140), die dafür konfiguriert ist, Folgendes zu empfangen: benutzerspezifische Informationen, die von einer Handvorrichtung (110) übertragen und spezifisch von einem Benutzer gespeichert werden, und biometrische Benutzerinformationen, die durch Messung der biometrischen Informationen des Benutzers erfasst werden,
wobei die Servervorrichtung (140) eine Zugriffspunkt-Informationsbestimmungseinheit (155) umfasst, die für Folgendes konfiguriert ist: Erhalten von Zugriffspunktinformationen, die einen Zugriffspunkt (130) darstellen, zu dem die Handvorrichtung (110) gegenwärtig gehört, und Bestimmen, ob der Zugriffspunkt geändert ist oder nicht;
eine Prognosebestimmungseinheit (158) für biometrische Informationen, die dafür konfiguriert ist, zu bestimmen, ob die biometrischen Benutzerinformationen ein vorbestimmtes Kriterium erfüllen oder nicht; und
die durch eine Spezifizierungseinheit (161) des Endgeräts der medizinischen Vorrichtung gekennzeichnet ist, die dafür konfiguriert ist, die benutzerspezifischen Informationen zu einem Endgerät (170) der medizinischen Vorrichtung zu übertragen, wenn bestimmt wird, dass sowohl der Zugriffspunkt geändert ist als auch das vorbestimmte Kriterium erfüllt ist.

10. Servervorrichtung nach Anspruch 9, die ferner Folgendes umfasst: eine Zugriffspunkt-Informationsspeichereinheit (152), die dafür konfiguriert ist, Zugriffspunktinformationen der Handvorrichtung (110) zu speichern,
wobei die Zugriffspunkt-Informationsbestimmungseinheit (155) dafür konfiguriert ist, zu bestimmen, dass der Zugriffspunkt (130), zu dem die Handvorrichtung (110) gehört, geändert ist, wenn sich die erworbenen Zugriffspunktinformationen von den Zugriffspunktinformationen, die in der Zugriffspunkt-Informationsspeichereinheit (152) gespeichert sind, unterscheiden.

11. Servervorrichtung nach Anspruch 9, die ferner Folgendes umfasst:

eine Erzeugungseinheit (156) für biometrische Informationsprofile, die dafür konfiguriert ist, ein benutzerspezifisches biometrisches Informationsprofil zu erzeugen, das auf vergangenen biometrischen Benutzerinformationen des Benutzers basiert, denen ein Fluktuationsmuster der vergangenen biometrischen Benutzerinformationen und ein Fluktuationswert von zukünftigen biometrischen Benutzerinformationen zugeordnet wird, wenn das Fluktuationsmuster auftritt; und
eine Fluktuationsmusterspezifizierungseinheit (157), die dafür konfiguriert ist, das Fluktuationsmuster der vergangenen biometrischen Benutzerinformationen zu spezifizieren, das mit einem Fluktuationsmuster der jüngsten biometrischen Benutzerinformationen des Benutzers des biometrischen Informationsprofils übereinstimmt, das von der biometrischen Informationsprofilerzeugungseinheit (156) erzeugt wird,

wobei die Prognosebestimmungseinheit (158) für biometrische Informationen ferner dafür konfiguriert ist, einen Prognosewert der zukünftigen biometrischen Benutzerinformationen zu berechnen, indem der Fluktuationswert der zukünftigen biometrischen Benutzerinformationen hinzugefügt wird, der dem Fluktuationsmuster der vergangenen biometrischen Benutzerinformationen zugeordnet ist, das durch die Fluktuationsmusterspezifizierungseinheit (157) spezifiziert wird, um biometrische Benutzerinformationen darzustellen und ferner dafür konfiguriert ist, zu bestimmen, unter Verwendung des Prognosewerts der zukünftigen biometrischen Benutzerinformationen, ob die biometrischen Benutzerinformationen das vorbestimmte Kriterium erfüllen oder nicht.

12. Servervorrichtung nach Anspruch 9, die ferner eine benutzerspezifische Informationslöscheinheit (160) umfasst, die dafür konfiguriert ist, die benutzerspezifischen Informationen zu löschen, die in dem Endgerät (170) der medizinischen Vorrichtung gespeichert sind, wenn die Zugriffspunkt-Informationsbestimmungseinheit (155) bestimmt, dass der Zugriffspunkt (130), zu dem die Handvorrichtung (110) gehört, geändert ist, nachdem die benutzerspezifischen Informationen zu dem Endgerät (170) der medizinischen Vorrichtung übertragen wurden.

**Revendications**

1. Système de fourniture d'informations spécifiques à un utilisateur (100) comportant un dispositif portable (110), un dispositif formant serveur (140) et un terminal d'installation médicale (170) ;

   le dispositif portable (110) étant configuré pour transmettre, au dispositif formant serveur (140), des informations spécifiques à l'utilisateur détenues spécifiquement par un utilisateur et des informations biométriques de l'utilisateur acquises par la mesure d'informations biométriques de l'utilisateur ;

   le dispositif formant serveur (140) étant configuré pour recevoir les informations spécifiques à l'utilisateur et les informations biométriques de l'utilisateur ; et

   le terminal d'installation médicale (170) étant configuré pour recevoir des informations transmises en provenance du dispositif formant serveur (140),

   le dispositif formant serveur (140) comprenant une unité de détermination (155) d'informations de point d'accès configurée pour acquérir des informations de point d'accès représentant un point d'accès (130) auquel le dispositif portable (110) appartient actuellement et pour déterminer si oui ou non le point d'accès (130) est changé,

   au moins l'un parmi le dispositif portable (110) et le dispositif formant serveur (140) comprenant par ailleurs une unité de détermination de prédiction d'informations biométriques configurée pour déterminer si oui ou non les informations biométriques de l'utilisateur satisfont à un critère prédéterminé,

   **caractérisé en ce que** le dispositif formant serveur (140) est configuré pour transmettre les informations spécifiques à l'utilisateur au terminal d'installation médicale (170) quand, à la fois, l'unité de détermination (155) d'informations de point d'accès détermine que le point d'accès est changé et l'unité de détermination de prédiction d'informations biométriques détermine que le critère prédéterminé est satisfait.

2. Système de fourniture d'informations spécifiques à un utilisateur selon la revendication 1, dans lequel le dispositif formant serveur (140) comprend par ailleurs une unité de stockage (152) d'informations de point d'accès configurée pour stocker des informations de point d'accès du dispositif portable (110), et

   l'unité de détermination (155) d'informations de point d'accès est configurée pour déterminer que le point d'accès (130) auquel le dispositif portable (110) appartient est changé quand les informations de point d'accès acquises sont différentes des informations de point d'accès stockées dans l'unité de stockage (152) d'informations de point d'accès.

3. Système de fourniture d'informations spécifiques à un utilisateur selon la revendication 1 ou la revendication 2, dans lequel au moins l'un parmi le dispositif portable (110) et le dispositif formant serveur (140) comprend par ailleurs :

   une unité de génération de profil (156) d'informations biométriques configurée pour générer un profil d'informations biométriques spécifiques à l'utilisateur, en fonction d'informations biométriques passées de l'utilisateur se rapportant à l'utilisateur, auquel sont associés un modèle de fluctuation des informations biométriques passées de l'utilisateur et une valeur de fluctuation de futures informations biométriques de l'utilisateur quand le modèle de fluctuation se produit ; et

   une unité de spécification (157) de modèle de fluctuation configurée pour spécifier le modèle de fluctuation des informations biométriques passées de l'utilisateur, qui coïncide avec un modèle de fluctuation d'informations biométriques récentes de l'utilisateur, parmi le profil d'informations biométriques généré par l'unité de génération de profil (156) d'informations biométriques, et

   l'unité de détermination de prédiction (158) d'informations biométriques est par ailleurs configurée pour calculer une valeur prédictive des futures informations biométriques de l'utilisateur par l'ajout de la valeur de fluctuation des futures informations biométriques de l'utilisateur, qui est associée au modèle de fluctuation des informations biométriques passées de l'utilisateur spécifié par l'unité de spécification (157) de modèle de fluctuation, aux informations spécifiques présentes de l'utilisateur, et est par ailleurs configurée pour déterminer si oui ou non les informations biométriques de l'utilisateur satisfont au critère prédéterminé par l'utilisation de la valeur prédictive des futures informations biométriques de l'utilisateur.

4. Système de fourniture d'informations spécifiques à un utilisateur selon la revendication 1, dans lequel le terminal d'installation médicale (170) comprend par ailleurs une unité de stockage (185) d'informations spécifiques à l'utilisateur configurée pour stocker les informations spécifiques à l'utilisateur ayant été transmises, et

   le dispositif formant serveur (140) est configuré pour effacer les informations spécifiques à l'utilisateur stockées dans l'unité de stockage (185) d'informations spécifiques à l'utilisateur du terminal d'installation médicale (170) quand l'unité de détermination (155) d'informations de point d'accès détermine que le point d'accès (130) auquel le dispositif portable (110) appartient est changé une fois que le dispositif formant serveur (140) a transmis les informations spécifiques à l'utilisateur au terminal d'installation médicale (170).

5. Procédé de fourniture d'informations spécifiques à un utilisateur exécuté par un dispositif formant serveur (140) en mesure de recevoir, en provenance d'un dispositif portable (110), des informations spécifiques à l'utilisateur détenues spécifiquement par un utilisateur et des informations biométriques de l'utilisateur acquises par la mesure d'informations biométriques de l'utilisateur, le dispositif formant serveur (140) recevant les informations spécifiques à l'utilisateur et les informations biométriques de l'utilisateur, le dispositif formant serveur (140) étant en mesure de transmettre des informations à un terminal d'installation médicale (170), le procédé comportant :

une étape (S702) du dispositif formant serveur (140) consistant à acquérir des informations de point d'accès représentant un point d'accès (130) auquel le dispositif portable (110) appartient actuellement et à déterminer si oui ou non le point d'accès (130) est changé ;
une étape (S705) de détermination de prédiction d'informations biométriques du dispositif formant serveur (140) consistant à déterminer si oui ou non les informations biométriques de l'utilisateur satisfont à un critère prédéterminé, ou du dispositif formant serveur (140) consistant à recevoir en provenance du dispositif portable (110) le résultat d'une détermination faite par le dispositif portable (110) quant à savoir si oui ou non les informations biométriques de l'utilisateur satisfont à un critère prédéterminé ;
une étape (S703) de détermination d'informations de point d'accès du dispositif formant serveur (140) consistant à déterminer si oui ou non le point d'accès (130) est changé ; et
une étape (S707) du dispositif formant serveur (140) consistant à transmettre les informations spécifiques à l'utilisateur au terminal d'installation médicale (170) quand il est déterminé qu'à la fois le critère prédéterminé est satisfait et le point d'accès (130) est changé.

6. Procédé de fourniture d'informations spécifiques à un utilisateur selon la revendication 5, dans lequel le dispositif formant serveur (140) comprend par ailleurs une unité de stockage (152) d'informations de point d'accès configurée pour stocker des informations de point d'accès du dispositif portable (110), et
l'étape de détermination d'informations de point d'accès détermine que le point d'accès (130) auquel le dispositif portable (110) appartient est changé quand les informations de point d'accès acquises sont différentes des informations de point d'accès stockées dans l'unité de stockage (152) d'informations de point d'accès.

7. Procédé de fourniture d'informations spécifiques à un utilisateur selon la revendication 5, dans lequel l'étape de détermination de prédiction d'informations biométriques comprend par ailleurs :

une étape de génération de profil d'informations biométriques consistant à générer un profil d'informations biométriques spécifiques à l'utilisateur, en fonction d'informations biométriques passées de l'utilisateur se rapportant à l'utilisateur, auquel sont associés un modèle de fluctuation des informations biométriques passées de l'utilisateur et une valeur de fluctuation de futures informations biométriques de l'utilisateur quand le modèle de fluctuation se produit ;
une étape de spécification de modèle de fluctuation consistant à spécifier le modèle de fluctuation des informations biométriques passées de l'utilisateur, qui coïncide avec un modèle de fluctuation d'informations biométriques récentes de l'utilisateur se rapportant à l'utilisateur, dans le profil d'informations biométriques généré au cours de l'étape de génération de profil d'informations biométriques ;
une étape consistant à calculer une valeur prédictive des futures informations biométriques de l'utilisateur par l'ajout de la valeur de fluctuation des futures informations biométriques de l'utilisateur quand le modèle de fluctuation des informations biométriques passées de l'utilisateur se produit, qui est spécifié au cours de l'étape de spécification de modèle de fluctuation, aux informations spécifiques présentes de l'utilisateur, et
une étape consistant à déterminer par l'utilisation de la valeur prédictive des futures informations biométriques de l'utilisateur si oui ou non les informations biométriques de l'utilisateur satisfont au critère prédéterminé.

8. Procédé de fourniture d'informations spécifiques à un utilisateur selon la revendication 5, dans lequel le terminal d'installation médicale (170) comprend par ailleurs une unité de stockage (185) d'informations spécifiques à l'utilisateur servant à stocker les informations spécifiques à l'utilisateur ayant été transmises, et
le procédé comporte :

une étape (S803) de détermination d'informations de point d'accès du dispositif formant serveur (140) consistant à déterminer que le point d'accès (130) auquel le dispositif portable (110) appartient est changé après l'étape consistant à transmettre les informations spécifiques à l'utilisateur au terminal d'installation médicale (170) ; et
une étape (S809) d'effacement d'informations spécifiques à l'utilisateur du dispositif formant serveur (140) consistant à effacer les informations spécifiques à l'utilisateur stockées dans l'unité de stockage (185) d'informations spécifiques à l'utilisateur du terminal d'installation médicale (170) quand il est déterminé que le point d'accès (130) est changé.

9. Dispositif formant serveur (140) configuré pour recevoir des informations spécifiques à un utilisateur transmises en provenance d'un dispositif portable (110) et détenues spécifiquement par un utilisateur et des informations biométriques de l'utilisateur acquises par la mesure d'informations biométriques de l'utilisateur, le dispositif formant serveur (140) comportant :

une unité de détermination (155) d'informations de point d'accès configurée pour acquérir des informations de point d'accès représentant un point d'accès (130) auquel le dispositif portable (110) appartient actuellement et pour déterminer si oui ou non le point d'accès est changé ;
une unité de détermination de prédiction (158) d'informations biométriques configurée pour déterminer si oui ou non les informations biométriques de l'utilisateur satisfont à un critère prédéterminé ; et
**caractérisé par** une unité de spécification (161) de terminal d'installation médicale configurée pour transmettre les informations spécifiques à l'utilisateur à un terminal d'installation médicale (170) quand il est déterminé qu'à la fois le point d'accès est changé et le critère prédéterminé est satisfait.

10. Dispositif formant serveur selon la revendication 9, comportant par ailleurs une unité de stockage (152) d'informations de point d'accès configurée pour stocker des informations de point d'accès du dispositif portable (110),
dans lequel l'unité de détermination (155) d'informations de point d'accès est configurée pour déterminer que le point d'accès (130) auquel le dispositif portable (110) appartient est changé quand les informations de point d'accès acquises sont différentes des informations de point d'accès stockées dans l'unité de stockage (152) d'informations de point d'accès.

11. Dispositif formant serveur selon la revendication 9, comportant par ailleurs :

une unité de génération de profil (156) d'informations biométriques configurée pour générer un profil d'informations biométriques spécifiques à l'utilisateur, en fonction d'informations biométriques passées de l'utilisateur se rapportant à l'utilisateur, auquel sont associés un modèle de fluctuation des informations biométriques passées de l'utilisateur et une valeur de fluctuation de futures informations biométriques de l'utilisateur quand le modèle de fluctuation se produit ; et
une unité de spécification (157) de modèle de fluctuation configurée pour spécifier le modèle de fluctuation des informations biométriques passées de l'utilisateur, qui coïncide avec un modèle de fluctuation d'informations biométriques récentes de l'utilisateur, parmi le profil d'informations biométriques généré par l'unité de génération de profil (156) d'informations biométriques,

dans lequel l'unité de détermination de prédiction (158) d'informations biométriques est par ailleurs configurée pour calculer une valeur prédictive des futures informations biométriques de l'utilisateur par l'ajout de la valeur de fluctuation des futures informations biométriques de l'utilisateur, qui est associée au modèle de fluctuation des informations biométriques passées de l'utilisateur spécifié par l'unité de spécification (157) de modèle de fluctuation, aux informations spécifiques présentes de l'utilisateur, et est par ailleurs configurée pour déterminer par l'utilisation de la valeur prédictive des futures informations biométriques de l'utilisateur si oui ou non les informations biométriques de l'utilisateur satisfont au critère prédéterminé.

12. Dispositif formant serveur selon la revendication 9, comportant par ailleurs une unité d'effacement (160) d'informations spécifiques à l'utilisateur configurée pour effacer les informations spécifiques à l'utilisateur stockées dans le terminal d'installation médicale (170) quand l'unité de détermination (155) d'informations de point d'accès détermine que le point d'accès (130) auquel le dispositif portable (110) appartient est changé après avoir transmis les informations spécifiques à l'utilisateur au terminal d'installation médicale (170).

## FIG. 1A

# FIG. 1B

110 HANDHELD DEVICE

EP 2 420 183 B1

# FIG. 1C

**140 SERVER DEVICE**

ROM 147

| USER-SPECIFIC INFORMATION UPDATING UNIT | 154 |
| ACCESS POINT INFORMATION DETERMINING UNIT | 155 |
| BIOMETRIC INFORMATION PROFILE GENERATION UNIT | 156 |
| FLUCTUATION PATTERN SPECIFYING UNIT | 157 |
| BIOMETRIC INFORMATION PREDICTION DETERMINING UNIT | 158 |
| USER'S MEDICAL EXAMINATION HISTORY CHECKING UNIT | 159 |
| USER-SPECIFIC INFORMATION ERASING UNIT | 160 |
| MEDICAL FACILITY TERMINAL SPECIFYING UNIT | 161 |

148 AUXILIARY STORAGE UNIT

150 USER-SPECIFIC INFORMATION STORAGE UNIT
151 MEDICAL FACILITY TERMINAL STORAGE UNIT
152 ACCESS POINT INFORMATION STORAGE UNIT
153 PROFILE GENERATION STORAGE UNIT

145 TRANSMISSION /RECEPTION UNIT
144 NCU  MODEM
143

141 CPU

142 RAM  EEPROM 146

CLOCK 149

EP 2 420 183 B1

# FIG. 1D

170 MEDICAL FACILITY TERMINAL

TRANSMISSION/RECEPTION UNIT
- NCU ～174
- MODEM
- 172
- 173

176 ROM
172 RAM
175 EEPROM

171 CPU

181 GATE ARRAY

INPUT UNIT
179

DISPLAY UNIT
180

RECORDING UNIT
182

CLOCK
178

177 AUXILIARY STORAGE UNIT
- MEDICAL FACILITY TERMINAL IDENTIFYING INFORMATION STORAGE UNIT ～183
- USER'S MEDICAL EXAMINATION HISTORY UNIT ～184
- USER-SPECIFIC INFORMATION STORAGE UNIT ～185

EP 2 420 183 B1

# FIG. 2A

EP 2 420 183 B1

| USER IDENTIFYING INFORMATION | UPDATE DATE/TIME | USER-SPECIFIC INFORMATION | | | |
|---|---|---|---|---|---|
| | | ALLERGY | ANAMNESIS | CHRONIC DISEASE | HAVE-BREAKFAST TIME |
| 0000AA1 | AUG. 21, 2009 16:00:00 | NOTHING | NOTHING | LUMBAGO | 7:30 |

| USER-SPECIFIC INFORMATION | | | | | |
|---|---|---|---|---|---|
| AMOUNT OF CARBOHYDRATE (BREAKFAST) | HAVE-LUNCH TIME | AMOUNT OF CARBOHYDRATE (LUNCH) | HAVE-SUPPER TIME | AMOUNT OF CARBOHYDRATE (SUPPER) | HOURS OF SLEEP YESTERDAY |
| 250g | NOTHING INGESTED | – | NOT YET INPUTTED | NOT YET INPUTTED | 8:00 |

# FIG. 2B

| USER IDENTIFYING INFORMATION | UPDATE DATE/TIME | ACCESS POINT INFORMATION |
|---|---|---|
| 0000AA1 | AUG. 21, 2009 16:00:00 | A |
| 0000AA2 | AUG. 21, 2009 16:00:00 | A |
| 0000AA2 | AUG. 21, 2009 16:00:00 | A |
| 0000AA3 | AUG. 21, 2009 16:00:00 | A |
| 0000AA3 | AUG. 21, 2009 16:00:00 | B |
| 0000AA4 | AUG. 21, 2009 16:00:00 | B |
| 0000AA4 | AUG. 21, 2009 16:00:00 | C |
| 0000AA5 | AUG. 21, 2009 16:00:00 | C |

## FIG. 2C

| USER IDENTIFYING INFORMATION | UPDATE DATE/TIME | USER-SPECIFIC INFORMATION | | | |
|---|---|---|---|---|---|
| | | ALLERGY | ANAMNESIS | CHRONIC DISEASE | HAVE-BREAKFAST TIME |
| 0000AA1 | AUG. 21, 2009 16:00:00 | NOTHING | NOTHING | LUMBAGO | 7:30 |
| 0000AA2 | AUG. 21, 2009 16:00:00 | NOODLE | NOTHING | HIGH BLOOD PRESSURE | 8:00 |
| 0000AA3 | AUG. 21, 2009 16:00:00 | WHEAT | NOTHING | NOTHING | 8:15 |
| 0000AA4 | AUG. 21, 2009 16:00:00 | NOTHING | BRONCHITIS | PODAGRA | 9:00 |
| 0000AA5 | AUG. 21, 2009 16:00:00 | NOTHING | HEPATITIS A | NOTHING | 6:00 |

| USER-SPECIFIC INFORMATION | | | | | |
|---|---|---|---|---|---|
| AMOUNT OF CARBOHYDRATE (BREAKFAST) | HAVE-LUNCH TIME | AMOUNT OF CARBOHYDRATE (LUNCH) | HAVE-SUPPER TIME | AMOUNT OF CARBOHYDRATE (SUPPER) | HOURS OF SLEEP YESTERDAY |
| 250g | NOTHING INGESTED | – | NOT YET INPUTTED | NOT YET INPUTTED | 8:00 |
| 100g | 12:45 | 100g | NOT YET INPUTTED | NOT YET INPUTTED | 7:30 |
| 300g | 13:00 | 200g | NOT YET INPUTTED | NOT YET INPUTTED | 5:00 |
| 80g | NOTHING INGESTED | – | NOT YET INPUTTED | NOT YET INPUTTED | 9:00 |
| 200g | 12:00 | 150g | NOT YET INPUTTED | NOT YET INPUTTED | 8:00 |

EP 2 420 183 B1

## *FIG. 2D*

| ACCESS POINT INFO. | MEDICAL FACILITY TERMINAL IDENTIFICATION INFO. |
|---|---|
| A | BB1@xxxx. co. jp |
| A | BB2@xxxy. co. jp |
| B | BB3@xxyy. co. jp |
| B | BB4@xyyy. co. jp |
| C | BB5@yyyy. co. jp |
| C | BB6@yyyz. co. jp |
| D | BB7@yyzz. co. jp |
| D | BB8@yzzz. co. jp |

## FIG. 2E

| FLUCTUATION PATTERN OF PAST BIOMETRIC INFORMATION | BLOOD GLUCOSE LEVEL DATA TRAIN SAMPLE CORRESPONDING TO PATTERN | FLUCTUATION VALUE OF BLOOD GLUCOSE LEVEL IN FUTURE FROM PAST TIME P |
|---|---|---|
| PATTERN 1 | BLOOD GLUCOSE LEVEL DATA TRAIN SAMPLE 1, BLOOD GLUCOSE LEVEL DATA TRAIN SAMPLE 16, BLOOD GLUCOSE LEVEL DATA TRAIN SAMPLE 10, ··· | −15.6 |
| PATTERN 2 | BLOOD GLUCOSE LEVEL DATA TRAIN SAMPLE 8, BLOOD GLUCOSE LEVEL DATA TRAIN SAMPLE 4, BLOOD GLUCOSE LEVEL DATA TRAIN SAMPLE 14, ··· | 26.5 |
| PATTERN 3 | BLOOD GLUCOSE LEVEL DATA TRAIN SAMPLE 18, BLOOD GLUCOSE LEVEL DATA TRAIN SAMPLE 17, BLOOD GLUCOSE LEVEL DATA TRAIN SAMPLE 3, ··· | −9.6 |
| PATTERN 4 | BLOOD GLUCOSE LEVEL DATA TRAIN SAMPLE 5, BLOOD GLUCOSE LEVEL DATA TRAIN SAMPLE 12, BLOOD GLUCOSE LEVEL DATA TRAIN SAMPLE 9, ··· | 22.4 |
| PATTERN 5 | BLOOD GLUCOSE LEVEL DATA TRAIN SAMPLE 7, BLOOD GLUCOSE LEVEL DATA TRAIN SAMPLE 2, BLOOD GLUCOSE LEVEL DATA TRAIN SAMPLE 13, ··· | 24 |
| PATTERN 6 | BLOOD GLUCOSE LEVEL DATA TRAIN SAMPLE 6, BLOOD GLUCOSE LEVEL DATA TRAIN SAMPLE 11, BLOOD GLUCOSE LEVEL DATA TRAIN SAMPLE 15, ··· | −12.5 |

EP 2 420 183 B1

## FIG. 2F

| USER IDENTIFYING INFORMATION | RECEPTION DATE/TIME | USER-SPECIFIC INFORMATION | | | |
|---|---|---|---|---|---|
| | | ALLERGY | ANAMNESIS | CHRONIC DISEASE | BREAKFAST TIME |
| 0000AA1 | AUG. 21, 2009 14:00:00 | NOTHING | NOTHING | LUMBAGO | 7:30 |
| 0000AA2 | AUG. 21, 2009 14:00:00 | NOODLE | NOTHING | HIGH BLOOD PRESSURE | 8:00 |

| USER-SPECIFIC INFORMATION | | | | | |
|---|---|---|---|---|---|
| AMOUNT OF CARBOHYDRATE (BREAKFAST) | LUNCH TIME | AMOUNT OF CARBOHYDRATE (LUNCH) | SUPPER TIME | AMOUNT OF CARBOHYDRATE (SUPPER) | HOURS OF SLEEP YESTERDAY |
| 250g | NOTHING INGESTED | – | NOT YET INPUTTED | NOT YET INPUTTED | 8:00 |
| 100g | 12:45 | 100g | NOT YET INPUTTED | NOT YET INPUTTED | 7:30 |

EP 2 420 183 B1

## *FIG. 2G*

| USER IDENTIFICATION INFO. | MEDICAL EXAMINATION DATE/TIME |
|---|---|
| 0000AA1 | AUG. 21, 2009  15:30:00 |
| 0000AA2 | AUG. 21, 2009  15:00:00 |

*FIG. 2H*

| MEDICAL FACILITY TERMINAL IDENTIFICATION INFOMATION | BB1@xxxx.co.jp | BB3@xxyy.co.jp | BB4@xyyy.co.jp | BB5@yyyy.co.jp | BB7@yyzz.co.jp |
|---|---|---|---|---|---|

## FIG. 3

```
            ( START )                    S301

  MEASURE BLOOD GLUCOSE LEVEL ON HANDHELD
  DEVICE                                   S302

  HANDHELD DEVICE TRANSMITS BLOOD GLUCOSE
  LEVEL DATA TO SERVER DEVICE

  SERVER DEVICE RECEIVES BLOOD GLUCOSE
  LEVEL DATA                            ~ S303

                                           S304
        PREDETERMINED TIME ELAPSED?          NO

                    YES                    S305

  DELIMIT INTO ONE BLOOD GLUCOSE LEVEL DATA
  TRAIN SAMPLE AND STORE SAMPLE IN RAM

                                           S306
          PREDETERMINED
        NUMBER OF BLOOD GLUCOSE
        LEVEL DATA TRAIN SAMPLES           NO
          ACCUMULATED?

                    YES                    S307

  CLASSIFY ACCUMULATED PAST BLOOD GLUCOSE
  LEVEL DATA TRAIN SAMPLES INTO USER'S
  PAST BIOMETRIC INFORMATION PATTERNS    S308

  CALCULATE FLUCTUATE VALUE OF USER'S
  PROSPECTIVE BIOMETRIC INFORMATION FROM
  PAST BLOOD GLUCOSE LEVEL DATA TRAIN SAMPLE
  CLASSIFIED INTO USER'S PAST BIOMETRIC
  INFORMATION PATTERNS, ASSOCIATE THE
  CALCULATED FLUCTUATION VALUE WITH PATTERN
  AND SET DATA AS USER-SPECIFIC BIOMETRIC
  INFORMATION PROFILE

                   ( END )
```

# FIG. 4

```
                    ( START )                    S401
                        │
                        ▼
┌─────────────────────────────────────────────┐
│ MEASURE BLOOD GLUCOSE LEVEL ON HANDHELD       │
│ DEVICE                                        │   S402
└─────────────────────────────────────────────┘
                        │
                        ▼
┌─────────────────────────────────────────────┐
│ HANDHELD DEVICE TRANSMITS BLOOD GLUCOSE       │
│ LEVEL DATA TO SERVER DEVICE                   │
└─────────────────────────────────────────────┘
                        │
                        ▼
┌─────────────────────────────────────────────┐
│ SERVER DEVICE RECEIVES BLOOD GLUCOSE          │   S403
│ LEVEL DATA                                    │
└─────────────────────────────────────────────┘
                        │
                        ▼              S404        NO
           < PREDETERMINED TIME ELAPSED? > ────────►
                        │
                        │ YES                       S405
                        ▼
┌─────────────────────────────────────────────┐
│ DELIMIT AS BLOOD GLUCOSE LEVEL DATA TRAIN     │
│ AND STORE DATA TRAIN IN RAM                   │   S406
└─────────────────────────────────────────────┘
                        │
                        ▼
┌─────────────────────────────────────────────┐
│ GENERATE FLUCTUATION PATTERS CLASSIFIED       │
│ WITH PREDETERMINED TIME ZONE BEFORE           │
│ PRESENT TIME                                  │
└─────────────────────────────────────────────┘
                        │
                        ▼              S407        NO
           < IS FLUCTUATION PATTERN           > ────────►
           < COINCIDENT WITH PROFILE?         >
                        │
                        │ YES                       S408
                        ▼
┌─────────────────────────────────────────────┐
│ CALCULATE PREDICTIVE VALUE OF PROSPECTIVE     │
│ BLOOD GLUCOSE LEVEL BY ADDING FLUCTUATION     │
│ VALUE OF PROSPECTIVE BLOOD GLUCOSE LEVEL      │
│ TO PRESENT BLOOD GLUCOSE LEVEL                │   S409
└─────────────────────────────────────────────┘
                        │
                        ▼
┌─────────────────────────────────────────────┐
│ DETERMINE WHETHER PREDICTIVE VALUE OF         │
│ PROSPECTIVE BLOOD GLUCOSE LEVEL IS EQUAL      │
│ TO OR SMALLER THAN FIRST PREDETERMINED        │
│ THRESHOLD VALUE OR EQUAL TO OR LARGER         │
│ THAN SECOND PREDETERMINED THRESHOLD VALUE     │
└─────────────────────────────────────────────┘
                        │
                        ▼
                    ( END )
```

# FIG. 5

|  | DEVIATION RATE | VARIATION RATE | DIFFERENCE |
|---|---|---|---|
| PATTERN 1 | POSITIVE | POSITIVE | POSITIVE |
| PATTERN 2 | POSITIVE | POSITIVE | NEGATIVE |
| PATTERN 3 | POSITIVE | NEGATIVE | POSITIVE |
| PATTERN 4 | NEGATIVE | POSITIVE | POSITIVE |
| PATTERN 5 | POSITIVE | NEGATIVE | POSITIVE |
| PATTERN 6 | NEGATIVE | NEGATIVE | NEGATIVE |

# FIG. 6

```
          ┌─────────────────┐
          (     START       )──────────  S601
          └────────┬────────┘
┌──────────────────▼──────────────────┐
│HANDHELD DEVICE TRANSMITS USER IDENTIFYING│
│INFORMATION AND USER-SPECIFIC INFORMATION │
│IN WAY OF BEING ASSOCIATED            │
└──────────────────┬──────────────────┘
                   ▼
            ╱──────────────╲
          ╱   USER-SPECIFIC  ╲         S602
        ╱  INFORMATION ≠ USER-SPECIFIC ╲    NO
        ╲  INFORMATION WITHIN SERVER   ╱──────┐
          ╲        DEVICE?           ╱        │
            ╲──────────────╱                  │
                   │ YES                 S603 │
┌──────────────────▼──────────────────┐      │
│UPDATE USER-SPECIFIC INFORMATION WITHIN│     │
│SERVER DEVICE                         │      │
└──────────────────┬──────────────────┘      │
                   ▼◄──────────────────────────┘
          ┌─────────────────┐
          (      END        )
          └─────────────────┘
```

EP 2 420 183 B1

## FIG. 7

START — S701

HANDHELD DEVICE TRANSMITS
USER IDENTIFYING INFORMATION ---- NOTHING

S702

SERVER DEVICE ACQUIRES ACCESS POINT
INFORMATION REPRESENTING ACCESS
POINT TO WHICH HANDHELD DEVICE
BELONGS AT PRESENT

USER IDENTIFYING
INFORMATION, ACCESS
POINT INFORMATION

USER IDENTIFYING
INFORMATION, ACCESS
POINT INFORMATION

S703
ANY CHANGE OF ACCESS POINT TO WHICH HANDHELD DEVICE BELONGS ? — NO

USER IDENTIFYING
INFORMATION, ACCESS
POINT INFORMATION

YES — S704

UPDATE DATABASE IN ACCESS POINT
INFORMATION STORAGE UNIT OF
SERVER DEVICE

USER IDENTIFYING
INFORMATION, ACCESS
POINT INFORMATION

S705
DOES BIOMETRIC INFORMATION PREDICTION DETERMINING UNIT DETERMINE THAT PREDETERMINED BENCHMARK IS SATISFIED ? — NO

USER IDENTIFYING
INFORMATION, ACCESS
POINT INFORMATION,
MEDICAL FACILITY
TERMINAL IDENTIFYING
INFORMATION

YES — S706

SERVER DEVICE SPECIFIES MEDICAL
FACILITY TERMINAL IN THE ACCESS
POINT TO WHICH HANDHELD DEVICE
BELONGS

USER IDENTIFYING
INFORMATION, MEDICAL
FACILITY TERMINAL
IDENTIFYING
INFORMATION,
USER-SPECIFIC
INFORMATION

S707

SERVER DEVICE TRANSMITS
USER-SPECIFIC INFORMATION TO
SPECIFIED MEDICAL FACILITY TERMINAL

USER IDENTIFYING
INFORMATION, MEDICAL
FACILITY TERMINAL
IDENTIFYING
INFORMATION

S708
CONTINUED TO STEP S801

END ---- NOTHING

42

# FIG. 8

```
              START
S801 ┌────────┴────────┐
     │ HANDHELD DEVICE TRANSMITS USER │
     │ IDENTIFYING INFORMATION AND    │        ┌ ─ ─ ─ ─ ─ ─ ─ ─ ─ ─ ┐
     │ PRESENT ACCESS POINT INFORMATION│─ ─ ─ ─ │ USER IDENTIFYING     │
     │ IN WAY OF BEING ASSOCIATED     │   S802  │ INFORMATION, MEDICAL │     ┌ ─ ─ ─ ─ ─ ─ ─ ─ ─ ─ ┐
     └────────┬────────┘              │         │ FACILITY TERMINAL    │     │ USER IDENTIFYING     │
     ┌────────┴────────┐              │         │ IDENTIFYING          │     │ INFORMATION, MEDICAL │
     │ SERVER DEVICE ACQUIRES ACCESS  │         │ INFORMATION          │     │ FACILITY TERMINAL    │
     │ POINT INFORMATION REPRESENTING │─ ─ ─     └ ─ ─ ─ ─ ─ ─ ─ ─ ─ ─ ┘     │ IDENTIFYING          │
     │ ACCESS POINT TO WHICH HANDHELD │     ─ ─ ─ ─ ─ ─ ─ ─ ─ ─ ─ ─ ─ ─ ─ ─ ─│ INFORMATION, ACCESS  │
     │ DEVICE BELONGS AT PRESENT      │         ┌ ─ ─ ─ ─ ─ ─ ─ ─ ─ ─ ┐     │ POINT INFORMATION    │
     └────────┬────────┘      S803    │         │ USER IDENTIFYING     │     └ ─ ─ ─ ─ ─ ─ ─ ─ ─ ─ ┘
```

FIG. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003057244 A
- JP 2005308742 A
- US 2005075116 A1
- JP 2004520898 A
- JP HEI0820412 B